(19) Europäisches Patentamt
European Patent Office
Office européen des brevets

(11) **EP 4 472 233 B1**

(12) **EUROPEAN PATENT SPECIFICATION**

(45) Date of publication and mention
of the grant of the patent:
**22.07.2026 Bulletin 2026/30**

(21) Application number: **22956354.9**

(22) Date of filing: **22.12.2022**

(51) International Patent Classification (IPC):
*H04R 1/10* (2026.01)          *A61B 5/00* (2006.01)
*A61B 5/107* (2006.01)        *A61B 5/11* (2006.01)
*G01L 9/08* (2006.01)          *G01L 11/04* (2006.01)
*G01L 27/00* (2006.01)        *G06F 3/01* (2006.01)
*H04R 25/00* (2006.01)        *G02B 27/01* (2006.01)

(52) Cooperative Patent Classification (CPC):
**G06F 3/014; A61B 5/1071; A61B 5/1125;
A61B 5/1126; A61B 5/6815; A61B 5/6885;
A61B 5/6898; G01L 9/08; G01L 11/04;
G01L 27/002; G06F 3/015; H04R 1/1041;
H04R 1/1091;** A61B 5/6806; A61B 5/6826;   (Cont.)

(86) International application number:
**PCT/CN2022/140963**

(87) International publication number:
**WO 2024/040821 (29.02.2024 Gazette 2024/09)**

(54) **ACOUSTIC OUTPUT APPARATUS COMPRISING AN EAR-HOOK**

AKUSTISCHES AUSGABEGERÄT MIT OHRBÜGEL

DISPOSITIF DE SORTIE ACOUSTIQUE COMPRENANT UN CROCHET D'OREILLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.08.2022   CN 202211006787**

(43) Date of publication of application:
**04.12.2024   Bulletin 2024/49**

(73) Proprietor: **Shenzhen Shokz Co., Ltd.
Shenzhen, Guangdong 518108 (CN)**

(72) Inventors:
 • **YUAN, Yongshuai**
  **Shenzhen, Guangdong 518108 (CN)**
 • **DENG, Wenjun**
  **Shenzhen, Guangdong 518108 (CN)**
 • **HUANG, Yujia**
  **Shenzhen, Guangdong 518108 (CN)**
 • **ZHOU, Wenbing**
  **Shenzhen, Guangdong 518108 (CN)**
 • **LIAO, Fengyun**
  **Shenzhen, Guangdong 518108 (CN)**
 • **QI, Xin**
  **Shenzhen, Guangdong 518108 (CN)**

(74) Representative: **Wang, Bo
Panovision IP
Ebersberger Straße 3
85570 Markt Schwaben (DE)**

(56) References cited:
  WO-A1-2016/011848       WO-A1-2016/011848
  WO-A1-2020/113375       WO-A1-2024/088113
  CN-A- 108 702 567       CN-A- 113 259 808
  CN-A- 113 259 808       CN-U- 211 128 122
  CN-U- 211 128 122       US-A1- 2013 083 933
  US-A1- 2018 199 127

(52) Cooperative Patent Classification (CPC): (Cont.)
A61B 2562/168; G02B 27/017; H04R 1/105;
H04R 25/603; H04R 25/607; H04R 2201/10;
H04R 2460/13

Description

CROSS-REFERENCE TO RELATED APPLICATIONS

This application claims priority to Chinese application No. 202211006787.3, filed August 22, 2022.

TECHNICAL FIELD

The present disclosure relates to the field of acoustics, and in particular, to acoustic output devices, in-ear headphones, and wearable devices.

BACKGROUND

In order to ensure a wearable experience for a user, a wearable device (e.g., an acoustic output device) in contact with the human body typically maintains a wearing pressure (or a wear pressure with the human body) in a wearing pressure range that matches the human body. Taking a bone-conducting headphone as an example, the wearing pressure not only affects the wearing comfort of the user, but also affects efficiency of transmission of a vibration signal output from the bone-conducting headphone to the human body. In addition, the wearing pressure is also a kind of feedback of a wearing position. Therefore, it is necessary to detect the wearing pressure of the acoustic output device. However, since a contact surface of the acoustic output device with the human body is often an irregular surface, and some acoustic output devices even have a flexible contact surface with the human body, conventional methods for detecting the wearing pressure is not applicable to the detection of the wearing pressure of the acoustic output device.
Therefore, it is desired to provide an acoustic output device capable of detecting the wearing pressure.
WO 2024/088113 A1 discloses an in-ear ear hook headset comprising an ear hook and an earplug, wherein the ear hook and the earplug are connected. When the user wears the headset, the earplug is inserted into the wearer's ear. The ear hook is hung on the wearer's ear and fits tightly to the wearer's skin just above the posterior auricular artery. A pressure detection device is located at the ear hook. CN 113 259 808 A relates to an intelligent Bluetooth earphone, comprising a housing, an ear hook, and an earplug. The ear hook can adapt to the auricles of different users through an auricle adjustment mechanism and a height adjustment mechanism. CN 211 128 122 U relates to an earphone comprising a body, on which a memory skeleton and a pressure sensor are mounted. The pressure sensor is used to collect the fitting pressure between the body and the skin, and the memory skeleton adjusts its own posture based on the data collected by the pressure sensor. US 2018/199127 A1 discloses an incoming/outgoing-talk unit having an ear-hook unit, a cartilage conduction vibration unit that makes contact with ear cartilage in a state where the hook unit is hooked to the ear, an outgoing-talk unit, and a unit for performing short-range wireless communication with the mobile phone. WO 2016/011848 A1 discloses a headphone capable of detecting heartrate.

SUMMARY

The invention is set out in the appended set of claims.

BRIEF DESCRIPTION OF THE DRAWINGS

FIG. 1 is a block diagram of an acoustic output device according to some embodiments of the present disclosure;
FIG. 2 is a schematic diagram of a calibration curve according to some embodiments of the present disclosure;
FIG. 3 is a schematic diagram of an exemplary structure of a sensing device according to some embodiments of the present disclosure;
FIG. 4 is a schematic diagram of an exemplary structure of a sensing device according to some embodiments of the present disclosure;
FIG. 5 is a schematic diagram of an exemplary structure of an acoustic output device according to some embodiments of the present disclosure;
FIG. 6 is a cross-sectional view of the ear hook at position A shown in FIG. 5;
FIG. 7 is a schematic diagram of an exemplary structure of an acoustic output device according to some other embodiments of the present disclosure;
FIG. 8 is a schematic diagram of an exemplary structure of an acoustic output device according to some other embodiments of the present disclosure;
FIG. 9 is a schematic diagram of an exemplary structure of an in-ear headphone according to some embodiments of the present disclosure;

FIG. 10A is a schematic diagram of an exemplary structure of an in-ear headphone according to some other embodiments of the present disclosure;

FIG. 10B is a schematic diagram of an exemplary structure of an in-ear headphone according to some other embodiments of the present disclosure;

FIG. 11A is a schematic diagram of an exemplary structure of an in-ear headphone according to some other embodiments of the present disclosure;

FIG. 11B is a schematic diagram of an exemplary structure of an in-ear headphone according to yet some other embodiments of the present disclosure;

FIG. 12 is a block diagram of a wearable device according to some embodiments of the present disclosure;

FIG. 13 is a schematic diagram of a structure of a smart bracelet according to some embodiments of the present disclosure;

FIG. 14 is a schematic diagram of a structure of an electrocardiogram (ECG) monitor according to some embodiments of the present disclosure;

FIG. 15 is a schematic diagram of a structure of glasses according to some embodiments of the present disclosure;

FIG. 16 is a schematic diagram of a structure of a virtual reality device or augmented reality device according to some embodiments of the present disclosure;

FIG. 17 is a schematic diagram of a structure of a smart ring according to some embodiments of the present disclosure; and

FIG. 18 is a schematic diagram of a curve of heart rate signals according to some embodiments of the present disclosure.

## DETAILED DESCRIPTION

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. Unless otherwise indicated or stated in the context, the same reference numerals in the drawings represent the same structures or operations.

It should be understood that the terms "system," "device," "unit" and/or "module" as used herein are a way to distinguish between different components, elements, parts, sections, or assemblies at different levels. However, these terms may be replaced by other words if other words accomplish the same purpose.

As indicated in the present disclosure and in the claims, the singular forms "a," "an," and "the" may be intended to include the plural forms as well, unless the context clearly indicates otherwise. In general, the terms "comprise," "comprises," and/or "comprising," "include," "includes," and/or "including," when used in this disclosure, specify the presence of stated features, integers, steps, operations, elements, and/or components, but do not preclude the presence or addition of one or more other features, integers, steps, operations, elements, components, and/or groups thereof.

Embodiments of the present disclosure describe an acoustic output device. In some embodiments, the acoustic output device includes an acoustic output unit and an ear hook, wherein the ear hook suspends the acoustic output unit near an ear of a user, and a sound signal output from the acoustic output unit outputs may be received by the user. The ear hook is provided with a volume-deformable flexible sealed cavity. When the user wears the acoustic output device, the flexible sealed cavity on the ear hook is in contact with the human body and deforms due a pressure resulting from contact with the human body, and it is possible to detect the pressure (hereinafter referred to as the wearing pressure, i.e., the pressure of the ear hook exerted on the human body when the user wears the acoustic output device) exerted by the user on the ear hook when the user wears the acoustic output device based on the deformation of the flexible sealed cavity. Further, an adjustment may be made based on the wearing pressure to improve an acoustic output effect of the acoustic output unit (e.g., a bone-conducting acoustic output unit) while improving the wearing comfort for the user. In addition, a wearing position of the acoustic output unit may be determined based on the wearing pressure when the user wears the acoustic output device, and then the wearing position of the acoustic output unit may be adjusted so that an acoustic conduction hole of the acoustic output unit (e.g., an air-conducting acoustic output unit) is oriented in a direction of an ear canal of the user, thereby improving the acoustic output effect of the acoustic output device.

In some embodiments, the acoustic output device may further include a sensing device configured to generate an electrical signal based on a change in the pressure within the flexible sealed cavity. In some embodiments, the sensing device may be provided in a location in the acoustic output device that is in contact with the user or in a structure (e.g., an ear hook, a rear-hanger, or a head beam) that is susceptible to deformation when the user wears the acoustic output device. The flexible sealed cavity on the ear hook may deform under the wearing pressure, and accordingly, a pressure of a fluid (e.g., a gas, a liquid, or a mix of a gas and a liquid) within the flexible sealed cavity changes, and the sensing device may generate an electrical signal in response to the change in the pressure. In some embodiments, there is a correspondence relationship between the electrical signal generated by the sensing device and the wearing pressure, and the wearing pressure may be determined based on the electrical signal. For example, a correspondence relationship curve (also referred to as a calibration curve) between the wearing pressure and the electrical signal may be obtained in advance, and the wearing pressure may be characterized by a preset load applied to the flexible sealed cavity during the obtaining process, and the correspondence relationship may be stored in the form of (a, A), wherein a denotes the preset load and A denotes an actually measured electrical signal when the load a is applied. Based on a plurality of correspondence relationships, a calibration curve may be fitted, and when the wearing pressure is detected, the wearing pressure may be determined based on the calibration curve and the electrical signal. The acoustic output device provided by embodiments of the present disclosure determines the wearing pressure, by using the flexible sealed cavity and based on the deformation of the flexible sealed cavity under the wearing pressure, the flexible sealed cavity can be applied to a wide range of contact surfaces (e.g., an irregular curved surface, a flexible curved surface, etc.) with the human body or structures (e.g., an ear-hook, a rear-hanger, or a head beam) that deform when the user wears the acoustic output device. In some embodiments, the contact surface of the flexible sealed cavity with the human body may be made of a flexible material, and the detection of the wearing pressure does not cause discomfort to the user, thereby balancing functionality and comfort. In some embodiments, a detection sensitivity of the acoustic output device may be adjusted by selecting different flexible materials and designing a shape and a size of the flexible sealed cavity to measure a tiny wearing pressure, so that the acoustic output device can also have a good sensitivity when detecting the tiny wearing pressure. In some embodiments, the sensing device may also identify, based on the detected pressure, whether or not the user has worn the acoustic output device to automatically trigger a command for controlling the acoustic output device, for example, to auto-play or auto-answer, etc.

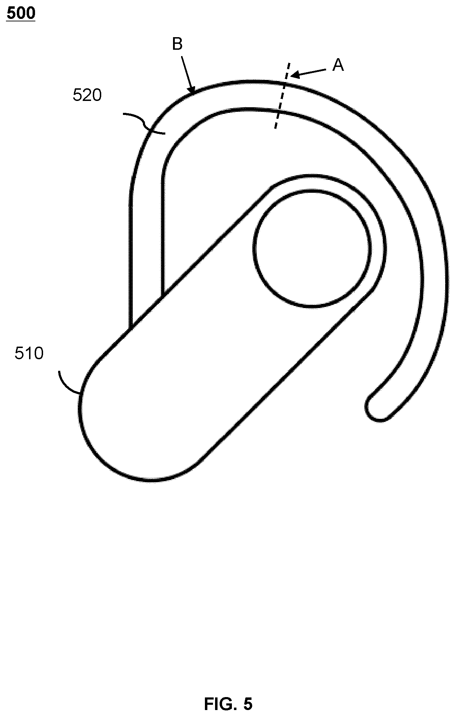

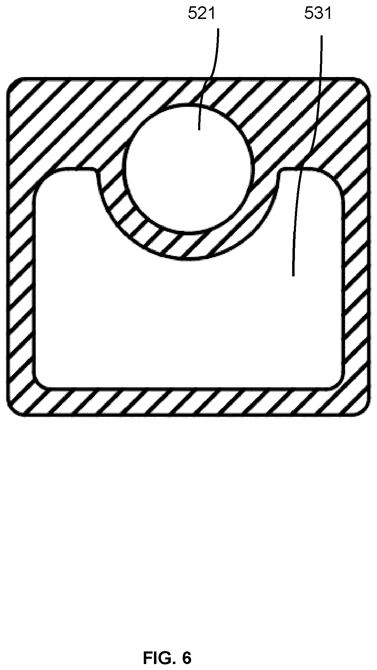

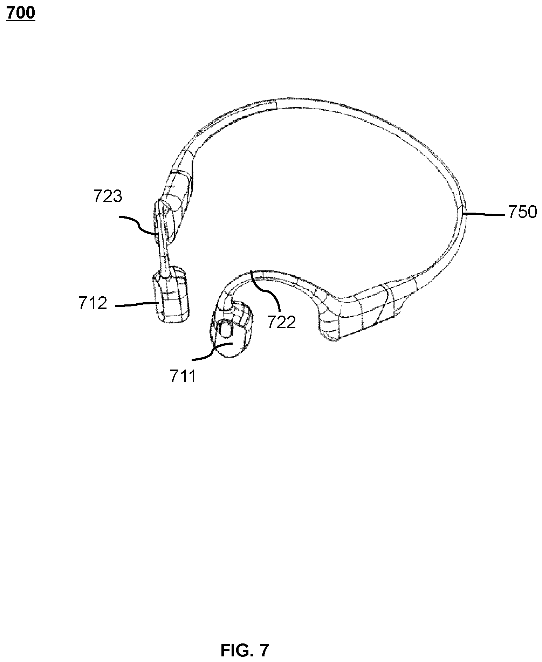

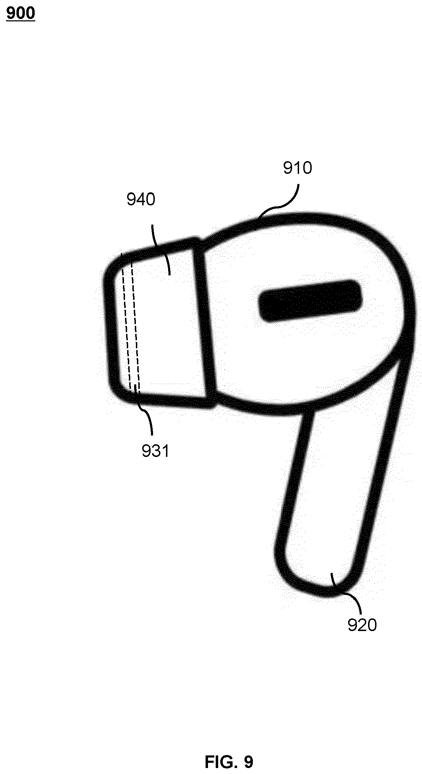

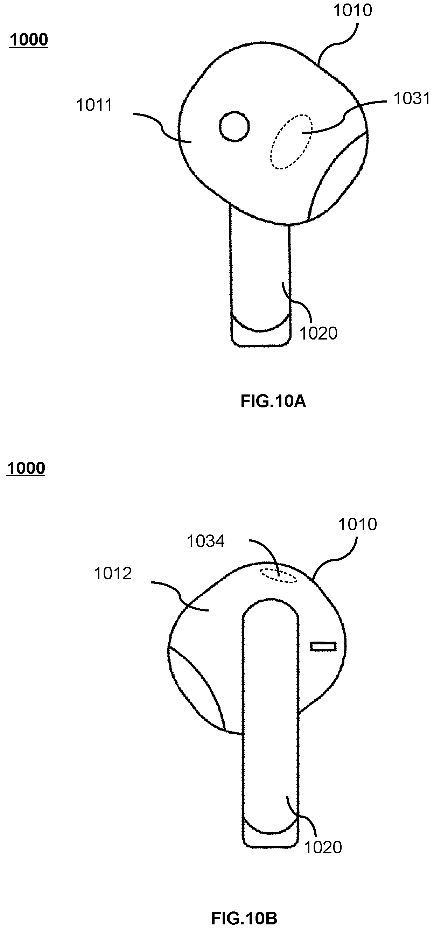

In some embodiments, the acoustic output device may include various types of acoustic devices, for example, a loudspeaker, a hearing aid, a bone-conducting headphone, an air-conducting headphone, a bone-air-conducting headphone, etc. In some embodiments, the acoustic output device may include a variety of headphones, for example, a hanging headphone (see FIGs. 5 and 6 and related depictions), a rear-hanging headphone (see FIG. 7 and related depictions), a headphone (see FIG. 7 and related depictions), an in-ear headphone (see FIG. 9 and related depictions), a semi-in-ear headphone (see FIGs. 10A-11B and related depictions), or the like.

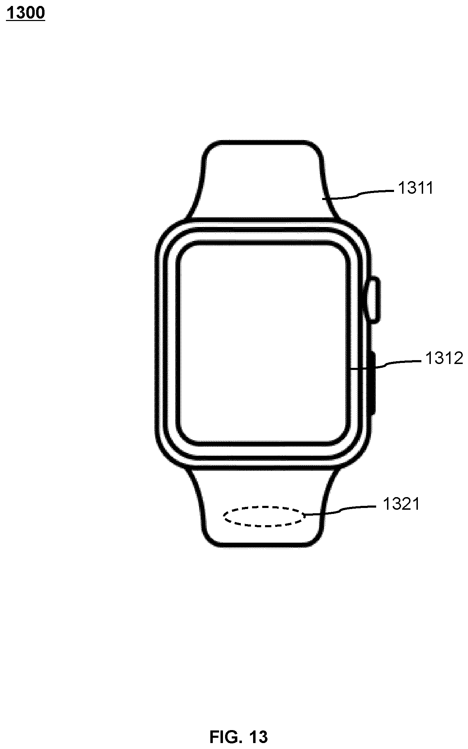

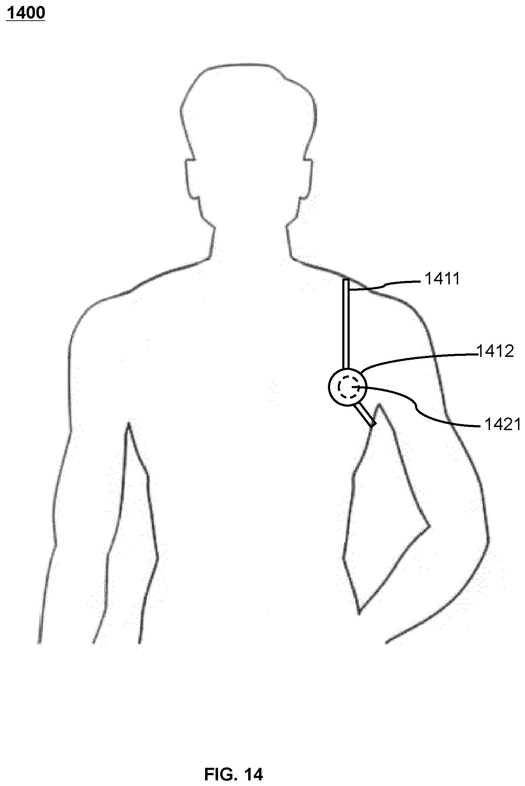

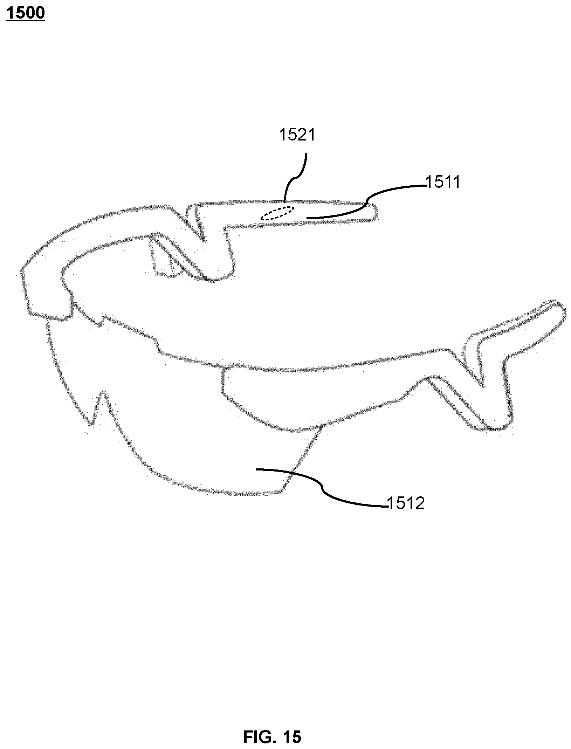

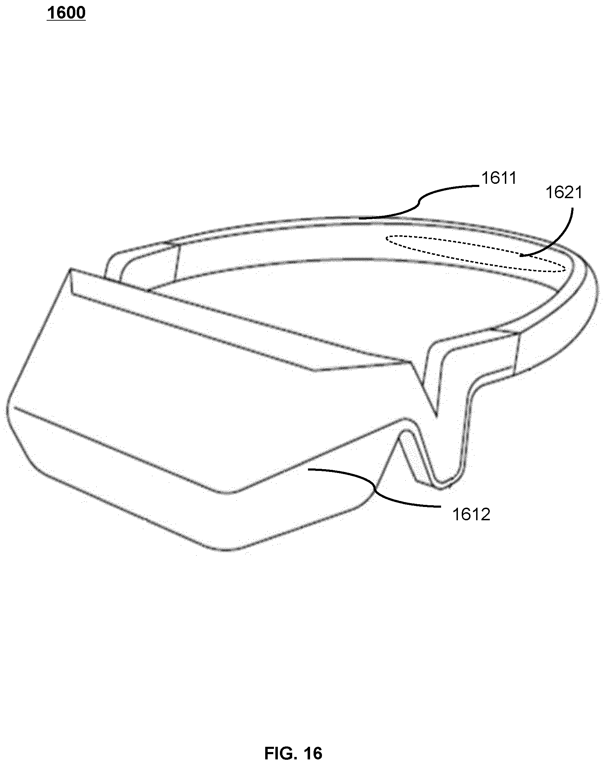

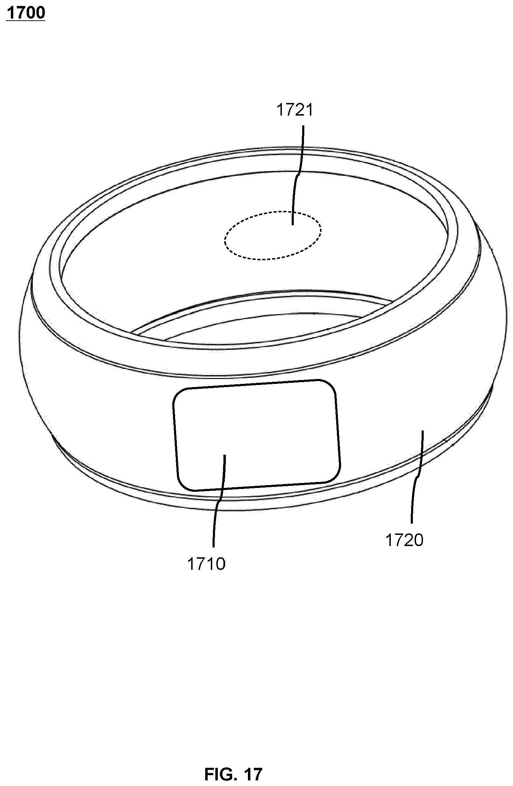

In some embodiments, the sensing device including the flexible sealed cavity may also be applied to various wearable devices to detect pressure exerted by the human body on the wearable device when the wearable device is worn on the human body. In some embodiments, the wearable device may be implemented as at least one of a smartwatch (see FIG. 13 and related depictions), an ECG monitor (see FIG. 14 and related depictions), glasses (see FIG. 15 and related depictions), a virtual reality (VR) device or augmented reality (AR) device (see FIG. 16 and related depictions), a smart ring (see FIG. 17 and related depictions), or the like. In some embodiments, the flexible sealed cavity on the wearable device is in contact with the human body, a physiological activity (e.g., heartbeat, respiration, pulse beat, etc.) of the user may cause deformation of the flexible sealed cavity, and the deformation may cause a change in the pressure inside the flexible sealed cavity. The sensing device may generate an electrical signal based on continuous changes in pressure, thereby obtaining a physiological signal based on the physiological activity of the user. In some embodiments, the physiological signal may include a pulse, a heart rate signal, a respiratory rate, or the like.

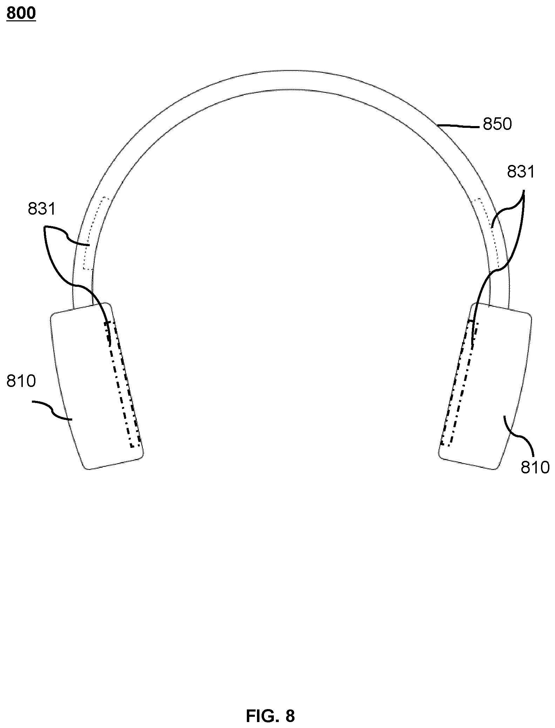

The acoustic output device, the in-ear headphone, and the wearable device provided by embodiments of the present disclosure will be exemplarily described below in connection with the accompanying drawings. Figures 8-14 and 15-17 do not fall within the scope of the appended claims.

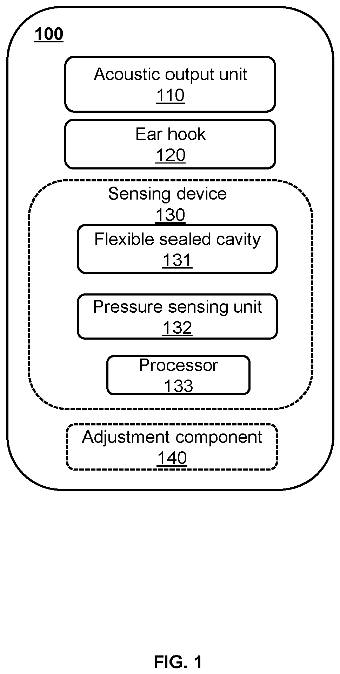

FIG. 1 is a block diagram of an acoustic output device according to some embodiments of the present disclosure.

As shown in FIG. 1, an acoustic output device 100 may include an acoustic output unit 110, an ear hook 120, and a sensing device 130.

The acoustic output device 100 refers to a device that converts an audio signal into sound. In some embodiments, the acoustic output device 100 may be applied to glasses, a smart bracelet, a headphone, a hearing aid, a smart helmet, a smart watch, a smart garment, a smart backpack, a smart accessory, etc., or any combination thereof. For example, the acoustic output device 100 may be applied to functional myopia glasses, presbyopia glasses, cycling glasses, sunglasses, etc., or may be applied to intelligent glasses, for example, audio glasses with a headphone function. In some embodiments, the acoustic output device 100 may also be applied to head-mounted devices such as a helmet, an Augmented Reality (AR) device, a Virtual Reality (VR) device, etc. In some embodiments, the AR device or the VR device may include a VR helmet, VR glasses, an AR helmet, AR glasses, etc., or any combination thereof. For example, the VR device and/or the AR device may include Google Glass, Oculus Rift, Hololens, Gear VR, or the like.

The acoustic output unit 110 may be configured to convert a signal containing acoustic information into an acoustic signal. In some embodiments, the acoustic output unit 110 may include one or more air-conducting speakers. In some embodiments, the acoustic output unit 110 may include one or more bone-conducting speakers. In some embodiments, the acoustic output unit 110 may include a combination of the one or more bone-conducting speakers and the one or more air-conducting speakers. In some embodiments, the acoustic output unit 110 may be connected to the ear hook 120, and when a user wears the acoustic output device 100, the ear hook 120 may secure the acoustic output unit 110 to an ear of the user or to an head region (e.g., a region anterior to the auricle, a region posterior to the auricle, a region superior to the auricle, etc.) near the ear in order to facilitate transmission of a sound emitted from the acoustic output device to the user. In some embodiments, the acoustic output unit 110 may be disposed at the end of the ear hook 120 or at any other location. For example, the acoustic output unit 110 may be provided only at an end of the ear hook 120 and other locations of the ear hook 120 are not provided with the acoustic output unit 110. In some embodiments, a plurality of acoustic output units 110 may be provided at a plurality of locations of the ear hook 120. For example, at least one acoustic output unit 110 may be provided at an end or other location(s) of the ear hook 120. In some embodiments, the acoustic output unit 110 may include a moving-coil acoustic output unit, an electrostatic acoustic output unit, a piezoelectric acoustic output unit, a moving-iron acoustic output unit, a pneumatic acoustic output unit, an electromagnetic acoustic output unit, or the like.

The ear hook 120 is configured to suspend the acoustic output unit 110 on an ear of the user or at a head region near the ear. In some embodiments, the ear hook 120 may be a structure that adapts to the ear of the user. For example, the ear hook 120 may be a curved structure that hangs above the ear. In some embodiments, the ear hook 120 may have a curved portion (see, for example, an ear hook 520 shown in FIG. 5), and the curved portion adapted to fit the ear may be configured to hang above the auricle of the ear of the user. In some embodiments, the ear hook 120 may be made of an elastic material, and exemplary elastic materials may include plastics, foams, rubbers, latex, silicone, sponges, metals, alloy materials, or the like, or any combination thereof. More description of the ear hook 120 may be found elsewhere in the present disclosure, such as in FIG. 5 and its related descriptions.

The sensing device 130 may be a sensing device configured to generate an electrical signal based on a change in the pressure. In some embodiments, the sensing device 130 may be provided in the ear hook 120. In some embodiments, the sensing device 130 may also be disposed at a location where the acoustic output unit 110 is in contact with the human body. In some embodiments, the sensing device 130 may be a pneumatic pressure sensing device, wherein the pneumatic pressure sensing device may deform under an external force and generate a change in air pressure within the pneumatic pressure sensing device, and furthermore, the pneumatic pressure sensing device may generate an electrical signal in response to the change in the air pressure. In some embodiments, the electrical signal generated by the sensing device 130 may be used to characterize a wearing pressure exerted on the sensing device 130. For example, there may be a correspondence relationship between the electrical signal generated by the sensing device 130 and the wearing pressure, e.g., the stronger the electrical signal is, the greater the wearing pressure may be. It should be noted that the sensing device 130 is not limited to the pneumatic pressure sensing device, but may also include a hydraulic sensing device, an air-hydraulic sensing device, or the like.

In some embodiments, the sensing device 130 may include a flexible sealed cavity 131, a pressure sensing unit 132, and a processor 133.

The flexible sealed cavity 131 may deform under an external load and produce a change in the pressure. In some embodiments, the flexible sealed cavity 131 may form a closed cavity, and a fluid may fill within the flexible sealed cavity 131. In some embodiments, the fluid may include a gas (e.g., air, nitrogen, etc.), a liquid (e.g., water), or a mixture of gas and liquid. Under an external load, the flexible sealed cavity 131 may deform to cause a change in a volume of the closed cavity, which causes the change in the pressure within the flexible sealed cavity 131. In some embodiments, the flexible sealed cavity 131 may be provided on the ear hook 120. For example, the flexible sealed cavity 131 may be disposed on the ear hook 120 near a top of an ear of the user. When the ear hook 120 is suspended on the ear of the user, the ear hook 120 made of an elastic material elastically deforms in a wearing state. The flexible sealed cavity 131 may be arranged in an extension direction along the ear hook 120, and the flexible sealed cavity 131 deforms at the same time as the ear hook 120 elastically deforms, which causes the change in the volume of the flexible sealed cavity 131, and thus the change in the pressure within the flexible sealed cavity 131.

Taking the liquid within the flexible sealed cavity 131 as air as an example, when the flexible sealed cavity 131 deforms and produces a change in air pressure, the change in air pressure of the flexible sealed cavity 131 may be related to the change in the volume of the flexible sealed cavity 131 due to the deformation of the flexible sealed cavity 131. The principle is as follows: when a gas is at equilibrium, a relationship between a pressure, a volume, and a temperature of the gas may be expressed by Equation (1):

$$pV = nRT \qquad\qquad (1)$$

where p denotes the pressure, unit in Pa, V denotes the volume of the gas, unit in $m^3$, T denotes the temperature, unit in K, n denotes an amount of substance of the gas, unit in mol, and R denotes a molar gas constant, unit in J/mol.K.

According to Equation (1), the relationship between the pressure and the volume of the gas may be further expressed by Equation (2) when the amount of substance of the gas is constant and the temperature is constant:

$$p_1 V_1 = p_2 V_2 \hspace{4cm} (2)$$

According to Equation (2), for the gas within the flexible sealed cavity 131, the volume of the flexible sealed cavity 131 is inversely proportional to the air pressure within the flexible sealed cavity 131. Accordingly, in conjunction with the description above, when the acoustic output device 100 is worn on the human body, the pressure of the human body with respect to the ear hook 120 and the flexible sealed cavity 131 causes the flexible sealed cavity 131 to deform, changing the volume of the flexible sealed cavity 131, thereby generating a change in air pressure within the flexible sealed cavity 131.

The pressure sensing unit 132 may be a sensor for detecting fluid pressure. In some embodiments, the pressure sensing unit 132 is in fluid communication with the flexible sealed cavity 131, and the pressure sensing unit 132 generates an electrical signal based on the change in the pressure within the flexible sealed cavity 131. In some embodiments, the pressure sensing unit 132 may be disposed in a closed cavity formed by the flexible sealed cavity 131, and the pressure sensing unit 132 may be configured to convert the change in the pressure into an electrical signal in response to detecting the change in the pressure of fluid within the closed cavity. For example, a thin-film structure may be disposed at an opening of the pressure sensing unit 132, and a change in the pressure within the flexible sealed cavity 131 may cause the thin-film structure to deform, thereby causing a change in the pressure within the pressure sensing unit 132. The pressure sensing unit 132 converts the change in the pressure within it into an electrical signal. In some embodiments, the pressure sensing unit 132 may include a sensing component (e.g., a MEMS sensor) and a thin-film structure. One end of the sensing component may have an opening, and the thin film structure covers the opening. A change in the pressure within the flexible sealed cavity 131 may deform the thin-film structure of the pressure sensing unit 132, thereby causing a change in the pressure within the sensing component, and the sensing component may convert the change in the pressure within it into an electrical signal. By providing the pressure sensing unit 132 inside the flexible sealed cavity 131, on the one hand, it is possible to enable the pressure sensing unit 132 to receive the change in the pressure inside the flexible sealed cavity 131 to generate an electrical signal; on the other hand, the flexible sealed cavity 131 can protect the pressure sensing unit 132 and its internal components (e.g., the sensing component) from being damaged.

The processor 133 identifies the wearing pressure based on the electrical signal. The wearing pressure may cause the flexible sealed cavity 131 to deform, and accordingly, the flexible sealed cavity 131 undergoes a change in the pressure, and the pressure sensing unit 132 in turn generates an electrical signal based on the change in the pressure. In some embodiments, there is a correspondence relationship between the electrical signal and the wearing pressure.

In some embodiments, the processor 133 may identify the wearing pressure by referencing a correspondence relationship table. In some embodiments, the wearing pressure may be identified based on a preset correspondence relationship table and an electrical signal. The correspondence relationship table may reflect the correspondence relationship between the electrical signal and the wearing pressure. Specifically, after the pressure sensing unit 132 generates an electrical signal, the wearing pressure corresponding to the electrical signal may be found in the preset correspondence relationship table based on the electrical signal. In some embodiments, the preset correspondence relationship table may be obtained by testing. In some embodiments, a correspondence relationship curve (also referred to as a calibration curve) between the wearing pressure and the electrical signal may be obtained in advance, and the calibration curve may be interpolated based on the electrical signal of the pressure sensing unit 132 to obtain the wearing pressure corresponding to the electrical signal. Exemplary operations of obtaining the calibration curve may be as follows. Different preset loads may be applied to the flexible sealed cavity 131, the preset loads characterizing wearing pressures, and electrical signals corresponding to the wearing pressures characterized by the preset loads may be measured, respectively. Then a calibration curve may be fitted based on a plurality of sets of preset loads and electrical signals.

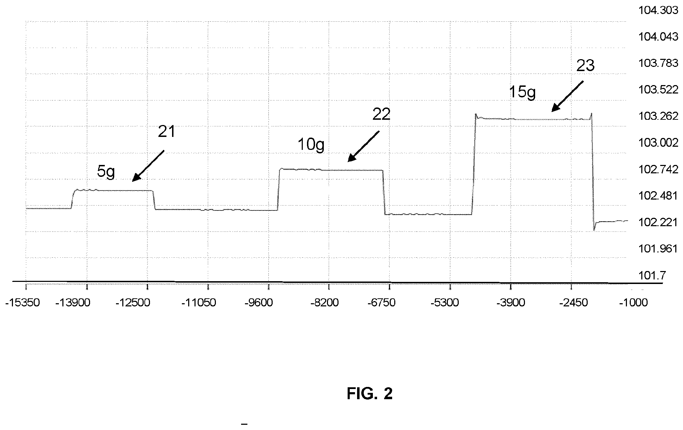

FIG. 2 is a schematic diagram of a calibration curve according to some embodiments of the present disclosure. Taking the fluid inside the flexible sealed cavity 131 as a gas and the pressure sensing unit as a gas sensor as an example, as shown in FIG. 2, the horizontal axis represents times during which preset loads are applied to the flexible sealed cavity 131, unit in milliseconds (ms), and the vertical axis represents values of air pressure (i.e., the electrical signal) of the pressure sensing unit 132, unit in kilopascals (kPa). The calibration curve shown in FIG. 2 illustrates three peaks, i.e., a peak 21, a peak 22, and a peak 23. The peak 21 represents the air pressure value of the pressure sensing unit 132 when the flexible sealed cavity 131 is under a 5g load, the peak 22 represents the air pressure value of the pressure sensing unit 132 when the flexible sealed cavity 131 is under a 10g load, and the peak 23 represents the air pressure value of the pressure sensing unit 132 when the flexible sealed cavity 131 is under a 15g load. As the load applied to the flexible sealed cavity 131 increases, the air pressure values corresponding to the peaks 21, 22, and 23 increase sequentially. Thus, it may be seen that a relationship between the preset load and the air pressure of the pressure sensing unit 132 is approximately linear. The larger the preset load is, the larger the air pressure of the pressure sensing unit 132 is; the smaller the preset load is, the smaller the air pressure of the pressure sensing unit 132 is. That is to say, there is an approximate positive linear relationship between the wearing pressure and the air pressure of the pressure sensing unit 132.

In some embodiments, the processor 133 may also identify the wearing pressure based on a machine learning model. An input of the machine learning model may be an electrical signal. An output of the machine learning model may be a wearing pressure. The machine learning model may be obtained by training an initial machine learning model (e.g., a neural network model) based on a plurality of training samples. In some embodiments, the training samples may include sample electrical signals and training labels. The training labels may be wearing pressures. In some embodiments, the training labels may be obtained from historical experimental data or obtained through manual labeling. In some embodiments, the machine learning model may include a deep neural network (DNN) model or a convolutional neural network (CNN) model.

In some embodiments, the processor 133 may be configured to determine a contact force between the acoustic output unit 110 and a skin of the user near the ear (e.g., the skin on the front, upper, lower, or back side of the auricle) based on the pressure. In some embodiments, the contact force between the acoustic output unit 110 and the skin of the user near the ear is correlated with the wearing pressure, and the contact force may be determined by the wearing pressure of the ear hook. For example, if the acoustic output device is an ear kook headphone (e.g., the acoustic output device 500 shown in FIG. 5), the contact force between the acoustic output unit 110 and the skin of the user near the ear is positively correlated with the wearing pressure of the ear hook. As another example, if the acoustic output device is a rear hook headphone (e.g., the acoustic output device 700 shown in FIG. 7), the acoustic output unit may include a first acoustic output unit and a second acoustic output unit, and the first acoustic output unit and the second acoustic output unit are located in a region adjacent to the left ear of the user and a region adjacent to the right ear of the user, respectively. The contact force between the first acoustic output unit and the skin of the user near the left ear is positively correlated with the wearing pressure of the ear hook to which the first acoustic output unit is connected, and the contact force between the second acoustic output unit and the skin of the user near the right ear is positively correlated with the wearing pressure of the ear hook to which the second acoustic output unit is connected. In some embodiments, in order to facilitate determination of a contact force between the acoustic output unit 110 and a facial region of the user, the wearing pressure of the ear hook when the user wears the acoustic output device 100 may be approximated as the contact force between the acoustic output unit and the facial region of the user. Exemplarily, when the user wears the acoustic output device 100, the wearing pressure of the ear hook is 0.2 **N,** and the contact force between the acoustic output unit and the facial region of the user is approximated as 0.2 N.

If the acoustic output unit is a bone-conducting acoustic output unit, the contact force between the bone-conducting acoustic output unit and the facial region of the user may affect sound transmission efficiency of the bone-conducting acoustic output unit. For example, if the contact force between the bone-conducting acoustic output unit and the facial region of the user is too small, the sound transmission efficiency of the bone-conducting acoustic output unit may be relatively low, thereby affecting the listening experience of the user. Factors causing the contact force between the bone-conducting acoustic output unit and the facial region of the user to be too small may include that the bone-conducting acoustic output unit is worn in an incorrect position when the user wears the acoustic output device. For example, the bone-conducting acoustic output unit needs to be placed in contact with an anterior side of the auricle in the facial region, however, the user places the bone-conducting acoustic output unit above or below the auricle in the facial region when wearing the acoustic output device. Factors causing the contact force between the bone-conducting acoustic output unit and the facial region of the user to be too small may also include variability in the body of the user, e.g., the user is young, has a small head size, etc. If the acoustic output unit is an air-conducted acoustic output unit, a wearing position of the air-conducted acoustic output unit may affect a position of an acoustic conduction hole with respect to an opening of an ear canal, and when the acoustic conduction hole is deviated from or facing away from the opening of the ear canal, a sound volume received by the user may be relatively low. Therefore, the position of the acoustic output unit, whether it is a bone-conducting or an air-conducting acoustic output unit, affects the listening experience of the user. Correspondingly, when a same user wears a particular acoustic output unit, if the wearing position of the acoustic output unit varies, the contact force between the acoustic output unit and the skin of the user near the ear may also vary. In some embodiments, the contact forces corresponding to the acoustic output unit when the acoustic output unit is at different wearing positions may be tested experimentally to create a database or a trained machine learning model that maps a relationship between the wearing position and the contact force. The wearing position of the acoustic output unit may then be determined based on the contact force and the database or the trained machine learning model, and the wearing position of the acoustic output device in the skin near the ear may be identified through the contact force. In some embodiments, in response to determining that the contact force is not within a preset contact force range, the processor may issue a control command to adjust the wearing position of the acoustic output unit such that the contact force is within the preset contact force range, or the processor may output a notification signal (e.g., a voice message, a light signal, or a beep signal, etc.) to the user to remind the user to adjust the wearing position of the acoustic output unit in the acoustic output device 100. Merely by way of example, if the contact force is not within the preset contact force range, the processor 133 may output a continuous beep to alert the user to adjust the wearing position of the acoustic output device 100, thereby ensuring better user comfort and sound transmission efficiency between the acoustic output unit and the user. It should be noted that when the user wears the acoustic output device 100, the wearing pressure of the ear hook is approximated to be the contact force between the acoustic output unit and the facial region of the user, and the contact force between the acoustic output unit and the skin of the user near the ear may be adjusted by adjusting the wearing pressure of the ear hook.

In some embodiments, the processor 133 may also be configured to identify whether or not the user is wearing the acoustic output device 100 based on the pressure. In some embodiments, the processor 133 may determine the pressure based on an electrical signal generated by the pressure sensing unit 132 and determine whether the pressure is greater than a preset pressure threshold to identify whether the user is wearing the acoustic output device 100. In some embodiments, in response to determining that the the pressure is greater than the preset pressure threshold, the processor 133 may determine that the user is fully wearing the acoustic output device 100. In some embodiments, after determining that the user is fully wearing the acoustic output device 100, the processor 133 may then respond to other processing processes of the acoustic output device 100, for example, answering a phone call, automatically playing music, or the like. In some embodiments, when the acoustic output device 100 is not worn by the user, no deformation is generated in the flexible sealed cavity 131, and then there is no electrical signal generated in the pressure sensing unit 132. Therefore, if the processor 133 does not receive an electrical signal, it indicates that the acoustic output device 100 is not worn by the user, and the processor 133 may respond to other processing processes of the acoustic output device 100, for example, hanging up a phone call, pausing a piece of music, adjusting a sound volume, or the like. In some embodiments, the preset pressure threshold may be 0.1 N. In some embodiments, the preset pressure threshold may be 0.2 N. In some embodiments, the preset pressure threshold may be 0.3 N. It should be noted that the preset pressure threshold is not limited to the above-described values, but may also be other values, which may be adjusted according to the acoustic effect, the wearing comfort, or the like, when the user wears the acoustic output device 100. Considering that the ear hook of the acoustic output device 100 may deform when the user operates the acoustic output device 100 when not wearing the acoustic output device 100, the pressure sensing unit 132 may also generate an electrical signal based on the deformation of the ear hook. To improve the accuracy of determining whether the user is wearing the acoustic output device 100, in some embodiments, determining whether the user is wearing the acoustic output device can include determining if the pressure is within the preset pressure range within a specific time range. When the user wears the acoustic output device, the device remains relatively fixed relative to the body of the user, resulting in minimal fluctuations in the wearing pressure. By analyzing the pressure over a specific time range, it is possible to avoid abnormal judgment results caused by accidental touches to the acoustic output device 100. For example, the specific time range may be greater than 2 seconds, greater than 3 seconds, or other time ranges. For example, the preset pressure range may be 0.1 N - 0.8 N, 0.2 N - 0.6 N, 0.3 N - 0.5 N, or other pressure ranges.

Referring to FIG. 1 again, in some embodiments, the acoustic output device 100 further includes an adjustment component 140, and the processor 133 is further configured to determine whether the pressure is within the preset pressure range and, in response determining that the pressure is not within the preset pressure range, the processor 133 controls the adjustment component 140 to adjust deformation of the ear hook 120, so that the pressure of the ear hook is within the preset pressure range.

In some embodiments, the adjustment component 140 may include a piezoelectric element, the piezoelectric element being a device in which voltage is positively correlated with a deformation of the piezoelectric element. The piezoelectric element may deform after receiving a driving voltage. In some embodiments, the piezoelectric element may be disposed on the ear hook 120 along an extension of the ear hook 120. For example, if the ear hook 120 includes an elastic metal wire and an elastic covering body wrapping the metal wire, the piezoelectric element may be attached to the elastic metal wire and covered by the elastic covering body. As another example, the piezoelectric element may be attached to a surface of the elastic covering body.

In some embodiments, the piezoelectric element may be made of a piezoelectric material that generates a voltage based on deformation. Exemplary piezoelectric materials may include piezoelectric ceramic, piezoelectric crystal, piezoelectric polymer (e.g., vinylidene fluoride), or the like, or any combination thereof. In some embodiments, the piezoelectric element may be in any shape, such as a sheet, a block, a column, a ring structure, etc., or any combination thereof. In some embodiments, the acoustic output device 100 may include a plurality of piezoelectric elements to enable shape adjustments at multiple locations of the ear hook 120.

In some embodiments, after the user wears the acoustic output device 100, the processor 133 may receive the electrical signal generated by the pressure sensing unit 132, determine the wearing pressure, and determine whether the pressure is within the preset pressure range. If the pressure is within the preset range, it indicates that the pressure between the ear hook 120 and the user is appropriate, or that the user's wearing method is correct, which means that the wearing position of the acoustic output unit on the skin near the ear is suitable, and the pressure is neither too loose nor too tight for the user or the application scenario. At this time, the acoustic output unit is correctly positioned on the skin near the ear of the user, allowing the acoustic output unit to transmit sound (e.g., bone-conducted sound or air-conducted sound) to the auditory nerves of the user with high transmission efficiency. If the pressure is not within the preset range, it indicates that the pressure is either too small or too large for the user, or that the wearing position of the acoustic output unit on the skin near the ear is incorrect. In response to determining that the pressure is not within the preset range, the processor 133 may output a control signal to the piezoelectric element, generating a driving pressure applied to the piezoelectric element to adjust a shape of the ear hook 120, thereby ensuring that the pressure provided by the ear hook 120 whose shape is adjusted falls back within the preset pressure range, and improving the comfort of wearing the acoustic output device 100 while ensuring the correct wearing position of the acoustic output unit on the skin of the user near the ear. For example, a bending angle of a bending portion of the ear hook 120 may be adjusted to adjust the pressure between the ear hook 120 and the ear of the user. When the ear hook 120 deforms, the acoustic output unit moves at the same time, causing a change in the wearing position of the acoustic output unit. For example, the acoustic conduction hole of the air-conducting acoustic output unit may be oriented toward the opening of the ear canal of the user by adjusting the deformation of the ear hook 120. As another example, the bone-conducting acoustic output unit may be adjusted to the skin near the ear on the anterior side of the auricle of the user by adjusting the deformation of the ear hook 120. In some embodiments, the preset pressure range may be 0.1 N-0.8 N. In some embodiments, the preset pressure range may be 0.2 N-0.6 N. In some embodiments, the preset pressure range may be 0.3 N-0.5 N.

In some embodiments, the acoustic output device 100 may include a micro motor, the micro motor being configured to drive an angular adjustment between the ear hook 120 and the acoustic output unit 110, thereby adjusting the pressure between the ear hook 120 and the ear of the user. Merely by way of example, the micro motor may drive a decrease in an angle between the ear hook 120 and the acoustic output unit 110, thereby increasing the pressure between the ear hook 120 and the ear of the user. Conversely, the micro motor may drive an increase in the angle between the ear hook 120 and the acoustic output unit 110, thereby decreasing the pressure between the ear hook 120 and the ear of the user. In some embodiments, the ear hook 120 and the acoustic output unit 110 may be rotatably connected through the adjustment component 140. The adjustment component 140 may be a gear drive member, a chain drive member, a belt drive member, a worm gear, helix, or the like. In some embodiments, the micro motor may be disposed on the ear hook 120 or on the acoustic output unit 110, and a power output end of the micro motor may be connected with the adjustment component 140. The micro motor may drive the adjustment component 140 to move, thereby realizing the angular adjustment between the ear hook 120 and the acoustic output unit 110.

In some embodiments, in response to determining that the wearing pressure is not within the preset pressure range, the processor 133 may output a control signal (e.g., a forward rotation signal or a reverse rotation signal) to act on the micro motor, and the micro motor operates to drive the adjustment component 140 into motion. In some embodiments, in response to determining that the wearing pressure is greater than the preset pressure range, the processor 133 may output the reversal rotation signal to act on the micro motor, and the micro motor rotates reversely to drive the movement of the adjustment component 140 to increase the angle, which reduces the pressure between the ear hook 120 and the ear of the user, so that the wearing pressure is within the preset pressure range, thereby enhancing the comfort of the user wearing the acoustic output unit 100. In some embodiments, in response to determining that the wearing pressure is smaller than the preset pressure range, the processor 133 may output the forward rotation signal to act on the micro motor, and the micro motor rotates forwardly to drive the adjustment component 140 to move to reduce the angle between the ear hook 120 and the acoustic output unit 110 to increase the pressure between the ear hook 120 and the ear of the user, so that the wearing pressure is within the preset pressure range, thereby enhancing the contact force between the user and the acoustic output unit 110. In some embodiments, the movement of the adjustment component 140 may also be achieved without providing the micro motor. Exemplarily, the user may manually adjust the angle between the acoustic output unit 110 and the ear hook 120, a degree of bending of the ear hook, or the like, to adjust the pressure between the ear hook 120 and the facial region of the user when the user wears the acoustic output device.

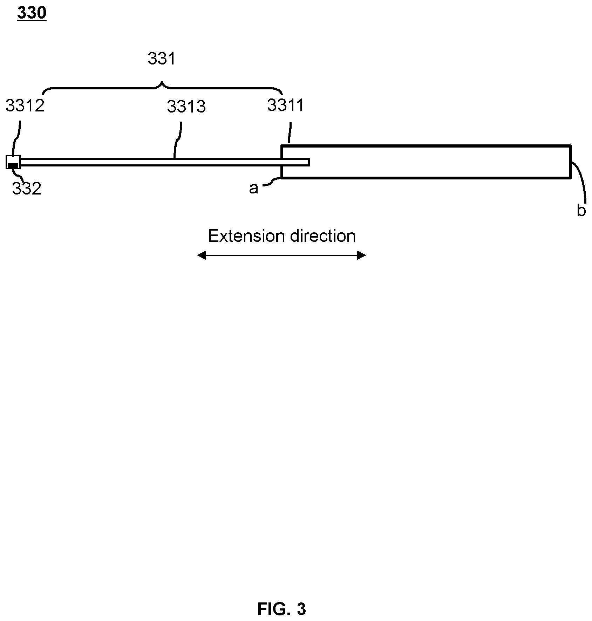

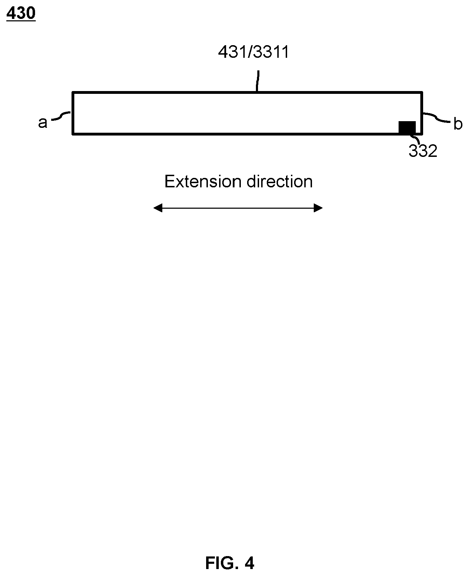

Further descriptions of the sensing device 130 may be found in FIG. 3 and FIG. 4 of the present disclosure, and the related descriptions thereof.

FIG. 3 is a schematic diagram of an exemplary structure of a sensing device according to some embodiments of the present disclosure. As shown in FIG. 3, a sensing device 330 may include a pressure sensing unit 332 and a flexible sealed cavity 331 in fluid communication with the pressure sensing unit 332. The fluid communication may refer to that a change in the pressure within the flexible sealed cavity 331 can be received by the pressure sensing unit 332. In some embodiments, the flexible sealed cavity 331 may deform and generate a change in the pressure within the flexible sealed cavity 331, and the pressure sensing unit 332 may generates an electrical signal in response to detecting the change in the pressure within the flexible sealed cavity 331.

In some embodiments, the flexible sealed cavity 331 may include a flexible cavity 3311, a connecting cavity 3312, and a connecting pipe 3313. The flexible cavity 3311, the connecting cavity 3312, and the connecting pipe 3313 may together form a closed cavity of the flexible sealed cavity 331. The flexible cavity 3311 may be configured to deform under an external load and produce a change in the pressure, the connecting cavity 3312 may be configured to accommodate the pressure sensing unit 332, and the connecting pipe 3313 may be configured to connect the flexible cavity 3311 and the connecting cavity 3312. In some embodiments, one end of the connecting pipe 3313 may be in fluid communication with the flexible cavity 3311, the other end of the connecting pipe 3313 in fluid communication with the connecting cavity 3312, and the change in the pressure within the flexible cavity 3311 may be transferred to the connecting cavity 3312 via the connecting pipe 3313. In some embodiments, an external load may act on (or primarily act on) the flexible cavity 3311, causing the flexible cavity 3311 to deform, resulting in a change in the pressure within the flexible cavity 3311. The change in the pressure within the flexible cavity 3311 is further transmitted to the connecting cavity 3312 through the connecting pipe 3313, so that the pressure sensing unit 332 may generate an electrical signal in respond to the change in the pressure within the flexible cavity 3311.

In some embodiments, to ensure that the flexible cavity 3311 has a good deformation effect under external loads, the flexible cavity 3311 may be made of a material that has good elasticity (i.e., a material that is susceptible to elastic deformation). In some embodiments, the material of the flexible cavity 3311 may be one or more of a polymer material, a gum material, or the like. In some embodiments, the polymeric material may be Polycarbonate (PC), Polyamides (PA), Acrylonitrile Butadiene Styrene (ABS), Polystyrene (PS), High Impact Polystyrene (HIPS), Polypropylene (PP), Polyethylene Terephthalate (PET), Polyvinyl Chloride (PVC), Polyurethanes (PU), Polyethylene (PE), Phenol Formaldehyde (PF), Urea-Formaldehyde (UF), Melamine-Formaldehyde (MF), Polyarylate (PAR), Polyetherimide (PEI), Polyimide (PI), Polyethylene Naphthalate two formic acid glycol ester (PEN), Polyetheretherketone (PEEK), silica gel, etc., or any combination thereof.

In some embodiments, the connecting pipe 3313 may be configured to connect the flexible cavity 3311 and the connecting cavity 3312, and the change in the pressure within the flexible cavity 3311 may be transmitted to the connecting cavity 3312 through the connecting pipe 3313, causing the pressure sensing unit 332 within the connecting cavity 3312 to generate the electrical signal in respond to the change in the pressure. In some embodiments, a relative position of the flexible cavity 3311 and the pressure sensing unit 332 may be adjusted by setting a connection position of the connection pipe 3313 and the flexible cavity 3311 so that the relative position of the flexible cavity 3311 and the pressure sensing unit 332 may be flexibly arranged, thereby enabling the sensing device 130 to adapt to different application scenarios.

In some embodiments, a material of the flexible cavity 3311 and a material of the connecting pipe 3313 may be of the same or different. In some embodiments, the external load acting on the flexible sealed cavity 331 that deforms the flexible cavity 3311 may also deform the connecting pipe 3313, and the deformation of the connecting pipe 3313 may result in a certain degree of (relatively small) change in the pressure, which may also be received by the pressure sensing unit 332 and converted into an electrical signal. Thus, in order to minimize an effect of the deformation of the connecting pipe 3313 under the external load on the electrical signal, the flexible cavity 3311 may be set to have a Young's modulus that is less than a Young's modulus of the connecting pipe 3313. In some embodiments, a ratio of the Young's modulus of the flexible cavity 3311 to the Young's modulus of the connecting pipe 3313 may be in a range of 1:1 to 1:10. In some embodiments, in order to minimize the effect of the deformation of the connecting pipe 3313 under the external load on the electrical signal, the ratio of the Young's modulus of the flexible cavity 3311 to the Young's modulus of the connecting pipe 3313 may be in a range of 1:3 to 1:8.

In some embodiments, a structure of the flexible cavity 3311 and/or a structure of the connecting pipe 3313 may be a tubular structure and/or an airbag structure. The tubular structure may include a square tube, a round tube, a vertebral tube, a curved tube, and other regular and/or irregular geometric structures. In some embodiments, a dimension (e.g., a length of the tubular structure, a diameter of the tubular structure, etc.) of the flexible cavity 3311 and/or the connecting tube 3313 may be reasonably set according to the actual application of the sensing device 330, e.g., a location of the sensing device 330 on a wearable device, and is not be limited herein.

In some embodiments, in order to minimize the effect of the deformation of the connecting pipe 3313 on the electrical signal, a diameter of the flexible cavity 3311 may be set to be larger than a diameter of the connecting pipe 3313. For example, when both the flexible cavity 3311 and the connecting pipe 3313 are round pipes, an end surface diameter of the flexible cavity 3311 may be larger than an end surface diameter of the connecting pipe 3313. In some embodiments, in order to minimize the effect of deformation of the connecting pipe 3313 on the electrical signal, a ratio of the diameter of the flexible cavity 3311 to the diameter of the connecting pipe 3313 may be greater than 3. By way of example only, the diameter (e.g., inner diameter) of the connecting pipe 3313 may be one-fourth of the diameter (e.g., inner diameter) of the flexible cavity 3311. It should be noted that when the flexible cavity 3311 or the connecting pipe 3313 is a round pipe, a cross-sectional area of the flexible cavity 3311 along a direction perpendicular to an extension direction of the flexible cavity 3311 may be greater than a cross-sectional area of the connecting pipe 3313 along a direction perpendicular to an extension direction of the connecting pipe 3313.

In some embodiments, the connecting cavity 3312 may be configured to accommodate the pressure sensing unit 332. The change in the pressure within the flexible cavity 3311 may be transmitted to the connecting cavity 3312 through the connecting pipe 3313, thereby causing a change of the pressure in the connecting cavity 3312, and the pressure sensing unit 332 may generate the electrical signal in response to detecting the change in the pressure within the connecting cavity 3312. As may be seen, a relationship between the change in the pressure within the flexible cavity 3311 and the change in the pressure within the connecting cavity 3312 may affect sensitivity of the pressure sensing unit 332. Taking the fluid inside the connecting cavity 3312 as a gas as an example, from Equations (1) and (2) above, for the gas within the connecting cavity 3312 (and/or the flexible cavity 3311), the volume of the connecting cavity 3312 (and/or the volume of the flexible cavity 3311) may be inversely proportional to the air pressure within the connecting cavity 3312 (and/or the air pressure within the flexible cavity 3311). As may be seen, the relationship between the volume of the flexible cavity 3311 and the volume of the connecting cavity 3312 may affect the sensitivity of the pressure sensing unit 332. Specifically, the smaller the volume of the connecting cavity 3312 is with respect to the volume of the flexible cavity 3311, the easier the change in the air pressure within the flexible cavity 3311 to cause a change in the air pressure within the connecting cavity 3312, and thus the higher the sensitivity of the pressure sensing unit 332 is. Thus, the sensitivity of the sensing device 130 may be adjusted (e.g., increased) by setting the volume of the connecting cavity 3312 and the volume of the flexible cavity 3311. In some embodiments, the volume of the connecting cavity 3312 may be smaller than the volume of the flexible cavity 3311 in order to increase the sensitivity of the sensing unit 130. In some embodiments, in order to increase the sensitivity of the sensing device 130, a ratio of the volume of the connecting cavity 3312 to the volume of the flexible cavity 3311 may not exceed 0.5. In some embodiments, to increase the sensitivity of the sensing device 130, the ratio of the volume of the connecting cavity 3312 to the volume of the flexible cavity 3311 may not exceed 0.1.

In some embodiments, when the pressure sensing unit 332 is disposed within the connectivity cavity 3312, the connectivity cavity 3312 may also protect the pressure sensing unit 332 and the internal components thereof. In order to enable the connecting cavity 3312 to act as a support to protect the pressure sensing unit 332, a material of the connecting cavity 3312 may be selected from a material with a relatively great hardness (e.g., a metal, an alloy, etc.). The material of the connecting cavity 3312 may be reasonably set according to actual situations (e.g., a location of the flexible sealed cavity 331 on the wearable device), and the embodiments of the present disclosure do not specifically limit this.

In some embodiments, the pressure sensing unit 332 may also be disposed in the flexible cavity 3311. FIG. 4 is a schematic diagram of an exemplary structure of a sensing device according to some embodiments of the present disclosure. The structure of a sensing device 430 shown in FIG. 4 is substantially the same as the structure of the sensing device 330 in FIG. 3, the difference is that a flexible sealed cavity 431 has a different structure. As shown in FIG. 4, the flexible sealed cavity 431 of the sensing device 430 includes the flexible cavity 3311. The flexible cavity 3311 may be a closed cavity. The pressure sensing unit 332 is disposed inside the flexible cavity 3311. Under the action of an external load, the flexible cavity 3311 may deform and produce a change in the pressure, and the pressure sensing unit 332 may convert the change in the pressure into an electrical signal. In some embodiments, the pressure sensing unit 332 may be located at any position (e.g., at an end, a middle, or other positions) of the flexible cavity 3311. In some embodiments, considering that in most cases, positions of the flexible cavity 3311 subject to pressure are non-end positions, in order to prevent the pressure sensing unit 332 from being damaged by pressure during use, the pressure sensing unit 332 may be located at one end of the flexible cavity 3311.

By providing the pressure sensing unit 332 inside the flexible cavity 3311, the structure of the flexible sealed cavity 331 may be simplified, and the effect of deformation of other structures (e.g., the connecting pipe) on the electrical signal may also be avoided.

Referring to FIG. 3 and FIG. 4, in some embodiments, the flexible cavity 3311 may have an extension direction. In some embodiments, the connecting cavity 3312 and the connecting pipe 3313 may be provided along the extension direction of the flexible cavity 3311. In some embodiments, the extension direction of the flexible cavity 3311 may be an axial direction of the flexible cavity 3311. In some embodiments, the flexible cavity 3311 may be coaxially disposed with the connecting cavity 3312 and the connecting pipe 3313. The extension direction of the flexible cavity 3311 may be parallel to a line connecting centers of two end surfaces of the flexible cavity 3311 (e.g., end surface a and end surface b shown in FIGs. 3 and 4). In some embodiments, if the flexible cavity 3311 is a non-uniform structure (e.g., a vertebral tubular structure), an external load acting on different positions of the flexible cavity 3311 may cause different positions of the flexible cavity 3311 to deform, which may result in different changes in the pressure generated by the flexible cavity 3311, and thus different electrical signals generated by the pressure sensing unit 332. The flexible cavity 3311 being a non-uniform structure may mean that cross-sectional areas of the flexible cavity 3311 perpendicular to the extension direction at different positions of the flexible cavity 3311 are different. For example, if the flexible cavity 3311 is a vertebral tubular structure, the cross-sectional area of the vertebral tubular structure perpendicular to the extension direction may gradually increase (or decrease) from one end of the vertebral tubular structure to the other end of the vertebral tubular structure. In some embodiments, in order to ensure that the pressure sensing unit 332 may generate substantially the same electrical signal when an external load is applied to different positions of the flexible cavity 3311, the flexible cavity 3311 may be configured as a homogeneous structure, i.e., the change in the cross-sectional area of the flexible cavity body 3311 perpendicular to the extension direction is within a suitable range. In some embodiments, in order to ensure that the pressure sensing unit 332 may generate the same or approximately the same electrical signal when an external load is applied to different positions of the flexible cavity 3311, the change in the cross-sectional area of the flexible cavity 3311 perpendicular to the extension direction of the cross section of the flexible cavity 3311 may not exceed 30%. For example, in order to ensure that the pressure sensing unit 332 may generate the same or approximately the same electrical signal when an external load is applied to different positions of the flexible cavity 3311, the change in the cross-sectional area of the flexible cavity 3311 perpendicular to the extension direction may be no more than 10%. In some embodiments, when the flexible cavity 3311 is placed on an acoustic output device to detect the wearing pressure of the ear hook on the ear, due to significant variations in the shape and size of ears of different users, the ear hook may not fit well with the ear of the user. In such cases, configuring the flexible cavity 3311 as a non-uniform structure allows the pressure sensing unit 332 to produce different electrical signals when the ear hook is worn on the ear and the wearing pressure is acted at different positions of the flexible chamber 3311, so that a position with a relatively high pressure may be identified based on the electrical signal, thereby facilitating an adjustment to the adjustment component (e.g., the piezoelectric element) at the position with the relatively high pressure.

In some embodiments, the ear hook has an extension direction, and when the flexible cavity 3311 is provided inside the ear hook, the extension direction of the flexible cavity 3311 is consistent with the extension direction of the ear hook, so as to allow the flexible cavity 3311 to sense the wearing pressure of the ear hook on the ear of the user based on the shape of the ear hook. In some embodiments, the extension direction of the ear hook may be an axial direction of the ear hook.

The acoustic output device provided in some embodiments is described in detail below in connection with FIG. 5 - FIG. 8.

FIG. 5 is a schematic diagram of an exemplary structure of an acoustic output device according to some embodiments of the present disclosure. As shown in FIG. 5, an acoustic output device 500 may include an acoustic output unit 510 and an ear hook 520, wherein the ear hook 520 is a curved structure adapted to a shape of an ear of a user, and one end of the ear hook 520 is connected to the acoustic output unit 510. When the user wears the acoustic output device 500, the ear hook 520 secures the acoustic output unit 510 near the ear of the user. In some embodiments, the acoustic output device 500 may further include a sensing device (not shown in FIG. 5) and an adjustment component (not shown in FIG. 5), and the manner in which the sensing device and the adjustment component are provided in the acoustic output device may be found in FIGs. 1, 3, and 4 and the related descriptions thereof. The connection manner described in the present disclosure may include a bolted connection, a riveted connection, an interference fit, snapping, bonding, injection molding, soldering, magnetic suction, or the like, or any combination thereof.

In some embodiments, the acoustic output unit 510 may be a bone-conducting speaker or an air-conducting speaker. In some embodiments, the acoustic output unit 510 may be a regular or irregular structural body such as a rectangular body, a cylindrical body, a rounded platform, an ellipsoidal body, a hemispherical body, a stepped platform, or the like. In some embodiments, the acoustic output unit 510 may include a contact surface that is in contact with the facial region or the ear of the use. When the user wears the acoustic output device 500, the contact surface of the acoustic output unit 510 may be in contact with a facial region near the ear of the user, positioned near the ear of the user, or partially extends into an ear canal of the user, thereby allowing the user to receive sound information output by the acoustic output unit 510. In some embodiments, the contact surface of the acoustic output unit 510 that is in contact with the facial region or the ear of the user may be a sidewall of the acoustic output unit 510. For example, if the acoustic output unit 510 is a cylinder, the contact surface may be a bottom surface or a side surface of the cylinder.

In some embodiments, the ear hook 520 may be a curved structure that fits around the ear (e.g., the auricle) of the user, and the curved structure that fits around the ear of the user may be configured to hang above the auricle of the ear of the user. In some embodiments, the ear hook 520 may include an elastic metal wire and an elastic covering body wrapping the metal wire. Further, a material of the elastic metal wire may include, but is not limited to, a spring steel, a titanium alloy, a titanium-nickel alloy, a chromium-molybdenum steel, a memory alloy, or the like, and a material of the elastic covering body may include, but is not limited to, polycarbonate, polyamide, silicone, rubber, etc.

In some embodiments, the ear hook 520 may also be provided with an accommodation cavity in which a linear structure and/or a wire that outputs power or a signal to the acoustic output unit 510 is threaded. The linear structure may be configured to define a shape of the ear hook 520, and make the shape of the ear hook 520 adjustable. In some embodiments, the linear structure may be an elastic metal wire. The user may adjust the shape of the ear hook 520 having the linear structure to enable the ear hook 520 to fit the ear, thereby ensuring comfort and stability of wearing the acoustic output device. In some embodiments, a piezoelectric element may be provided on the linear structure, and the shape adjustment of the wire structure may be realized by adjusting a shape of the piezoelectric element by a processor.

FIG. 6 is a cross-sectional view of the ear hook at position A shown in FIG. 5 according to some other embodiments of the present disclosure. Referring to FIG. 6, the ear hook 520 is provided with an accommodation cavity 521 and a flexible sealed cavity 531, and the accommodation cavity 521 is juxtaposed with the flexible sealed cavity 531. The flexible sealed cavity 531 is similar to the flexible cavity 331 shown in FIG.3 and the flexible cavity 431 shown in FIG.4.

In some embodiments, the accommodation cavity 521 and the flexible sealed cavity 531 may be provided along an extension direction of the ear hook 520. In some embodiments, a linear structure (not shown in FIG. 6) may be connected to an inner wall of the accommodation cavity 521. In some embodiments, diameters at various positions of the linear structure within the accommodation cavity 521 may be different to stabilize the structure and shape of the ear hook 520 and to enhance the durability of the ear hook 520. For example, the diameter of the linear structure near a top of the auricle of the ear (at position B shown in FIG. 5) may be relatively large, and the diameter of the linear structure at a position near the lobe of the ear (i.e., an end on the ear hook 520 that is away from the acoustic output unit 510) may be relatively small. When adjusting the shape of the ear hook 520, the end of the ear hook 520 that is away from the acoustic output unit 510 may be rotated by using the position near the top of the auricle as a pivot point to make the end of the ear hook 520 that is away from the acoustic output unit 510 fit with the auricle. As another example, the diameter of the linear structure may be relatively large at a position near the top of the auricle of the ear, and the diameter of the linear structure may be relatively small at a position near the ear canal (i.e., an end of the ear hook 520 on which the acoustic output unit 510 is provided). When adjusting the shape of the ear hook 520, the end of the ear hook 520 near the acoustic output unit 510 may be rotated with the position near the top of the auricle of the human ear 520 as a pivot point to move the acoustic output unit 510 near the ear canal. In some embodiments, the diameter of the linear structure at the position near the top of the auricle of the ear may be set to be relatively large, thereby preventing a portion of the linear structure near the top of the auricle of the ear from being severely damaged during repeated adjustments of the shape of the ear hook 520. In some embodiments, the shape of the accommodation cavity 521 may be adapted to the linear structure. It should be noted that the cross-sectional shape of the accommodation cavity 521 is not limited to a circle as shown in FIG. 6, but may also be a regular or irregular shape such as a triangle, a trapezoid, a rectangle, a diamond, or the like.

In some embodiments, the accommodation cavity 521 and the flexible sealed cavity 531 may form an integral structure on the ear hook 520. In some embodiments, the accommodation cavity 521 and the flexible sealed cavity 531 may form a closed cavity in which the linear structure and the pressure sensing unit are provided.

FIG. 7 is a schematic diagram of an exemplary structure of an acoustic output device according to some other embodiments of the present disclosure. As shown in FIG. 7, an acoustic output device 700 may further include a rear hook 750, an ear hook of the acoustic output device 700 may include a first ear hook 722 and a second ear hook 723, and an acoustic output unit of the acoustic output device 700 may include a first acoustic output unit 711 and a second acoustic output unit 712. The first acoustic output unit 711 may be connected to an end of the rear hook 750 via the first ear hook 722, and the second ear hook 723 may be connected to another end of the rear hook 750 via the second acoustic output unit 712. The first ear hook 722 may suspend the first acoustic output unit 711 near one ear of a user, the second ear hook 723 may suspend the second acoustic output unit 712 near the other ear of the user, and the first ear hook 722 and the second ear hook 723 may be connected via the rear hook 750. The first ear hook 722 and the second ear hook 723 are similar to the ear hook 520 shown in FIG. 5, and the first acoustic output unit 711 and the second acoustic output unit 712 are similar to the acoustic output unit 510 shown in FIG. 5.

In some embodiments, the rear hook 750 may have a curved shape, and when the user wears the acoustic output unit 700, the rear hook 750 may be suspended on a back side of the head of the user, the first ear hook 722 and the second ear hook 723 may be set on the two ears of the user, respectively, and the first acoustic output unit 711 and the second acoustic output unit 712 may be provided near the two ears of the user, respectively. For example, the first acoustic output unit 711 and the second acoustic output unit 712 may be located in facial regions on front sides of the two ears of the user, respectively. In some embodiments, the first acoustic output unit 711 and the second acoustic output unit 712 may be secured at positions near the two ears of the user without blocking the ear canals of the user. For example, if the first acoustic output unit 711 and the second acoustic output unit 712 are bone-conducting speakers or hearing aids, bone-conducting sound waves generated by the bone-conducting speakers or the hearing aids may be transmitted to the user hearing through the bones, blood, muscles, or the like of the user to the auditory nerve of the user. As another example, the first acoustic output unit 711 and the second acoustic output unit 712 may be open air-conducting speakers with sound outlets that may be oriented in directions towards the ear canals of the user, respectively. In some embodiments, in order to adapt to the shape of the back side of the head of different users or different requirements of the user for wearing tightness in different application scenarios, the rear hook 750 may have an elasticity, and the rear hook 750 may produce different deformation when different users wear the acoustic output device.

In some embodiments, a sensing device may also be provided on the rear hook 750, and the sensing device may be provided on the rear hang 750 in a similar manner as it is provided on the ear hook. In some embodiments, the sensing device may include a flexible sealed cavity extending along an extension direction of the rear hook 750. When the user wears the acoustic output device 700, the wearing pressure may cause the flexible sealed cavity to deform, and accordingly the deformation causes a change in the pressure in the flexible sealed cavity. The pressure sensing unit may generate an electrical signal based on the change in the pressure, and the processor may identify the wearing pressure based on the electrical signal. In some embodiments, the flexible sealed cavity may be disposed only in a midsection of the rear hook 750. When the user wears the acoustic output device 700 shown in FIG. 7, the flexible sealed cavity may be located at a position on the rear hook 750 near the back side of the head of the user. The pressure at the position on the rear hook 750 near the back side of the head of the user may be relatively high, therefore, only detecting the pressure at this location may accomplish the purpose of detecting the wearing pressure. In some embodiments, the flexible sealed cavity may include a plurality of flexible sealed cavities, the plurality of flexible sealed cavities may be configured to detect pressures at different positions, and different preset pressure ranges may be set for the pressures at the different positions, thereby achieving precise adjustment of the pressures at the different positions. For example, the flexible sealed cavity may include a first flexible sealed cavity and a second flexible sealed cavity, the first flexible sealed cavity may be located at a position on the rear hook 750 close to a center point of the rear hook 750, and the second flexible sealed cavity may be located at a position on the rear hook 750 close to the mastoid process of the temporal bone of the user.

In some embodiments, an adjustment component may also be provided on the rear hook 750, and the adjustment component may be provided on the rear hook 750 in a similar manner as it is provided on the ear hook. For example, a piezoelectric element may be provided on the rear hook 750. As another example, the rear hook 750 may be rotatably connected with the ear hook via the adjustment component.

In some embodiments, the first acoustic output unit 711 and/or the second acoustic output unit 712 (hereinafter referred to as the acoustic output unit) may also be provided with a sensing device, and the sensing device may be provided on the acoustic output unit in a similar manner as it is provided on the ear hook. When the user wears the acoustic output device 700, the wearing pressure may cause the flexible sealed cavity to deform, and the deformation of the flexible sealed cavity may cause a change in the pressure within the flexible sealed cavity. The pressure sensing unit may generate an electrical signal based on the change in the pressure within the flexible sealed cavity, and the processor may identify the wearing pressure based on the electric signal. In some embodiments, the flexible sealed cavity may be disposed on the acoustic output unit on a side close to the facial region of the user, so that the flexible sealed cavity may accurately sense the pressure between the acoustic output unit and the face of the user. In some embodiments, the flexible sealed cavity may be disposed on a side of the acoustic output unit in front of an ear concha of the user, where the acoustic output unit has a relatively high pressure.

FIG. 8 is a schematic diagram of an exemplary structure of an acoustic output device according to some other embodiments of the present disclosure. As shown in FIG. 8, an acoustic output device 800 may include two acoustic output units 810 and a head beam 850, the head beam 850 may be a head-hanging structure adapted to be disposed on a top region of the head of a user, and two ends of the head beam 850 is connected to the two acoustic output units 810, respectively. The head beam 850 may be provided with a sensing device including a flexible sealed cavity 831. When the user wears the acoustic output device 800, the head beam 850 may be suspended at the top region of the head of the user, and the two acoustic output units 810 may cover the two ears of the user under an act of the head beam 850.

In some embodiments, a count of flexible sealed cavity 831 may be one or more. For example, if the count of flexible sealed cavity 831 is one, the one flexible sealed cavity 831 may be located at a center point position of the header beam 850 where the pressure is relatively high. For example, if the count of flexible sealed cavity 831 is two, the two flexible sealed cavities 831 may be located at positions on the head beam 850 near the two ears respectively where the pressures are relatively high. In some embodiments, the count of flexible sealed cavity 831 may be three or even more, and the specific manner of setting thereof may be referred to in FIG. 7 and its related description.

In some embodiments, one or more sensing devices may also be provided on the acoustic output unit 810. For example, if the acoustic output unit 810 is a cylindrical structure, the one or more flexible sealed cavities 831 of the one or more sensing devices may be distributed along an edge region of a bottom surface or an edge region of a side surface of the cylindrical structure, wherein the bottom surface or the side surface may be in contact with the user. As another example, the one or more flexible sealed cavities 831 of the one or more sensing devices may be located on the bottom surface of the cylindrical structure that is in contact with the user. The specific manner in which the one or more sensing devices are set up may be found in FIG. 7 and its related description. In some embodiments, the acoustic output unit 810 may be provided with an elastic pad on a side near the ear, and the elastic pad may be in direct contact with the face of the user. The one or more flexible sealed cavities 831 may be provided to detect a pressure between the elastic pad and the user.

The in-ear headphones provided in the present disclosure are described in conjunction with FIG. 9 - FIG.11B.

FIG. 9 is a schematic diagram of an exemplary structure of an in-ear headphone according to some embodiments of the present disclosure. As shown in FIG. 9, an in-ear headphone 900 may include a headphone body 910, a headphone handle 920, and a sensing device. The headphone handle 920 may be connected to the headphone body 910, and the headphone body 910 may be worn at an entrance of the ear canal of a user. The sensing device may be integrated inside the headphone 900, and the sensing device may include a flexible sealed cavity 931, a pressure sensing unit, and a processor. The flexible sealed cavity 931 may be provided at a position on the headphone body 910 where the headphone body 910 is in contact with the ear canal of the user, and the flexible sealed cavity 931 may be configured to detect a pressure exerted on the headphone body 910 when the user wears the in-ear headphone 900. The processor may be configured to determine whether the user is wearing the in-ear headphone 900 based on the detected pressure, and thereafter respond to other processing processes of an acoustic output device, such as answering a phone call, playing music automatically, or the like. More descriptions of the sensing device may be found in FIG. 1, FIG. 3, FIG. 4, and the related descriptions thereof.

In some embodiments, a front end of the headphone body 910, i.e., an end of the headphone body 910 near the ear canal of the user, may be provided with an elastic ring 940 (e.g., a silicone ring). The elastic ring 940 may be configured to wrap a sound outlet of the headphone body 910 and extend into the ear canal of the user. A sound signal output from the headphone body 910 passes through the elastic ring 940 to enter the ear canal, which is conducive to reducing a leakage of sound from the headphone 900. The elastic ring 940 may have an elasticity, which may allow the elastic ring 940 to adapt to a wide range of ear canal sizes of different users, and enhance the comfort of wearing the in-ear headphone 900. In some embodiments, the flexible sealed cavity 931 may be disposed in the elastic ring 940, and the user wearing the headset 900 may detect the pressure exerted by the human body on the elastic ring 940. In some embodiments, the flexible sealed cavity 931 may be integrally formed with the elastic ring 940, that is to say, a structure of the elastic ring 940 is provided with the flexible sealed cavity. In some embodiments, the flexible sealed cavity 931 may also be a separate structure with respect to the elastic ring 940, e.g., the flexible sealed cavity 913 may be affixed to an outer ring side or an inner ring side of the elastic ring 940. In some embodiments, the flexible sealed cavity 931 may be an annular structure disposed in a circumferential direction along a circumference of the elastic ring 940. In some embodiments, the flexible sealed cavity 931 may also be a rectangular structure, a spherical structure, a columnar structure, or the like, and the flexible sealed cavity 931 may be disposed in a localized region of the elastic ring 940. In some embodiments, the flexible sealed cavity 931 may be provided close to a front end (an end of the elastic ring 940 that extends into the entrance of the ear canal) of the elastic ring 940. The front end of the elastic ring 940 extends into the entrance of the ear canal of the user, which undergoes a relatively large elastic deformation, and accordingly, a shape of the flexible sealed cavity 931 also undergoes a relatively large change to improve the sensitivity of the sensing device. When the user wears the in-ear headphone 900, the flexible sealed cavity 931 on the elastic ring 940 may be contact with a wall of the ear canal and undergoes deformation, and the pressure sensing unit may generate an electrical signal in response to detecting the deformation. The processor may determine a pressure exerted on the elastic ring 940 based on the electrical signal. If the pressure is too small, the in-ear headphone 900 is easy to fall off, and a sound leakage reduction effect is not good. If the pressure is too large, the elastic ring 940 extends into the ear canal too much, which is detrimental to the hearing system of the user. Therefore, the sensing device may be provided on the in-ear headphone 900, which is conducive to determining the wearing pressure of the in-ear headphone 900 with respect to the human body, so as to facilitate adjustment of the wearing pressure to a suitable range.

FIG. 10A is a schematic diagram of an exemplary structure of an in-ear headphone according to some other embodiments of the present disclosure. FIG. 10B is a schematic diagram of an exemplary structure of an in-ear headphone according to some other embodiments of the present disclosure. As shown in FIGs. 10A and 10B, a headphone 1000 may include a headphone body 1010, a headphone handle 1020, and a sensing device. The headphone handle 1020 may be connected to the headphone body 1010, and the headphone body 1010 may be configured as a flat circular structure and worn at a concha of a user. For example, when the user wears the headphone 1000, a first side 1011 of the headphone body 1010 may cover and block an entrance of the ear canal of the user, the first side 1011 may be in contact with a region near the entrance of the ear canal, and a second side 1012 of the headphone body 1010 may be in contact with a tragus of the user. In some embodiments, the sensing device may be integrated into the headphone 1000, and the sensing device may be located at the first side 1011 and/or the second side 1012 of the headphone body 1010. In some embodiments, the sensing device may include one or more flexible sealed cavities. When the one or more flexible sealed cavities include one flexible sealed cavity, the one flexible sealed cavity may be disposed on either the first side 1011 or the second side 1012 of the headphone body 1010. Taking the sensing device including a plurality of flexible sealed cavities as an example, the plurality of flexible sealed cavities may include a first flexible sealed cavity 1031 and a second flexible sealed cavity 1034, and, as illustrated in FIG. 10A, the first flexible sealed cavity 1031 is provided on the first side 1011 of the headphone body 1010 for covering and blocking the entrance of the ear canal of the user, as shown in FIG.10B, and the second flexible sealed cavity 1034 is provided on the second side 1012 of the headphone body 1010 that is in contact with the tragus of the user. By providing the flexible sealed cavities on both the first side 1011 and the second side 1012 of the headphone body 1010, the wearing pressure of the headphone 1000 against the ear can be effectively detected, which can prevent misalignment and incorrect measurements. It should be noted that a plurality of first flexible sealed cavities 1031 may be provided on the first side 1011, and a plurality of second flexible sealed cavities 1034 may be provided on the second side 1012. More descriptions of the sensing device may be found in FIG. 1, FIG. 3, FIG. 4, FIG. 9, and the related descriptions thereof.

In some embodiments, the headphone body 1010 may include a housing, and the flexible sealed cavity (e.g., the first flexible sealed cavity 1031 or the second flexible sealed cavity 1034) may be configured as a closed flexible structure. For example, the closed flexible sealed cavity may be formed by a closed silicone air bladder, and the closed flexible structure may be disposed on an outer surface of the housing. In some embodiments, the closed flexible structure may be embedded in an outer side of the housing. In some embodiments, the closed flexible structure may protrude from a surface of the housing. In some embodiments, a portion of the closed flexible structure may be a portion of a surface region of the headphone 1000. In some embodiments, the closed flexible structure may also be integrated or seamlessly connected with other regions of the surface of the headphone 1000.

In some embodiments, a groove may be provided on an outer surface of the housing, a flexible structure (e.g., a silicone layer) may be provided at an opening of the groove, and the flexible structure may cover the opening and form the flexible sealed cavity (e.g., the first flexible sealed cavity 1031 or the second flexible sealed cavity 1034) with the groove. In some embodiments, a circumferential side of the flexible structure may be connected to a circumferential side of the groove to enclose the groove and form the flexible sealed cavity. In some embodiments, an inner side of the flexible structure may be connected to the groove, and the circumferential side of the flexible structure may be connected with the outer surface of the housing, thereby forming the flexible sealed cavity and forming a completely enclosed outer surface of the housing.

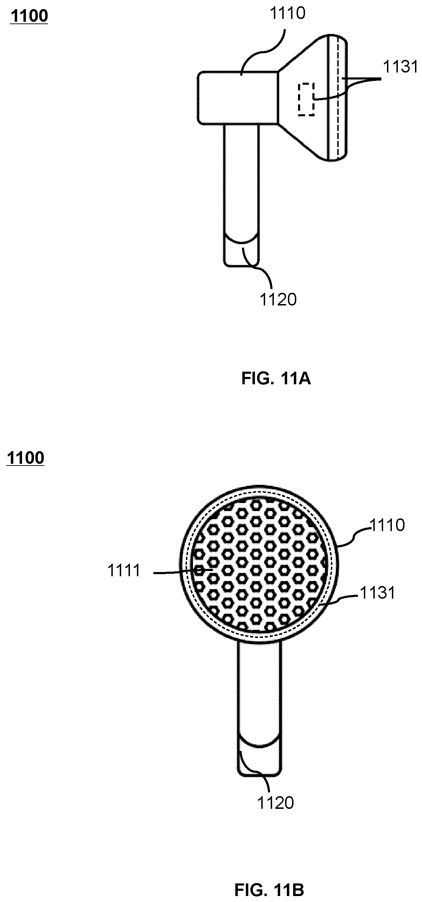

FIG. 11A is a schematic diagram of an exemplary structure of an in-ear headphone according to some other embodiments of the present disclosure. FIG. 11 B is a schematic diagram of an exemplary structure of an in-ear headphone according to yet some other embodiments of the present disclosure. As shown in FIG. 11A and FIG. 11 B, a headphone 1100 may include a headphone body 1110, a headphone handle 1120, and a sensing device. The headphone handle 1120 may be connected to the headphone body 1110. The headphone body 1110 may be configured as a trumpet-shaped structure and may be worn at a concha of a user, and one or more sound outlets 1111 may be provided on a side where a trumpet opening of the trumpet-shaped headphone body 1110 is located. When the user wears the headphone 1100, the side where the trumpet opening of the headphone body 1119 is located may cover and block an entrance of an ear canal of the user, and the one or more sound outlets 1111 may be oriented toward the ear canal of the user. The sensing device may be integrated inside the headphone 1100, and the sensing device may include a flexible sealed cavity 1131. In some embodiments, the flexible sealed cavity 1131 may be configured as an annular structure that wraps around the one or more sound outlets 1111 along a circumferential direction of the side of a trumpet opening of the trumpet-shaped headphone body 1110 to detect the pressure between the sound outlet 1111 and the concha, which is conducive to timely adjustment of the pressure, and also prevents sound signal leakage from a gap between the one or more sound outlets 1111 and the concha. In some embodiments, the flexible sealed cavity 1131 may also be configured as a structure of other shapes, for example, a spherical structure, a cylindrical structure, an ellipsoidal structure, or the like, and a count of the flexible sealed cavity 1131 may be more than one. A plurality of flexible sealed cavities 1131 may be spaced apart along an edge of a circumferential side of the trumpet opening of the headphone body 1110. In some embodiments, the flexible sealed cavity 1131 may also be disposed on a sidewall of the headphone body 1110, i.e., a sidewall of the trumpet-shaped structure, so that when the user wears the headphone 1100, the flexible sealed cavity 1131 may be in contact with the tragus of the user. More descriptions of the sensing device may be found in FIG. 1, FIG. 3, FIG .4, FIGs.9 -10 B, and the related descriptions thereof.

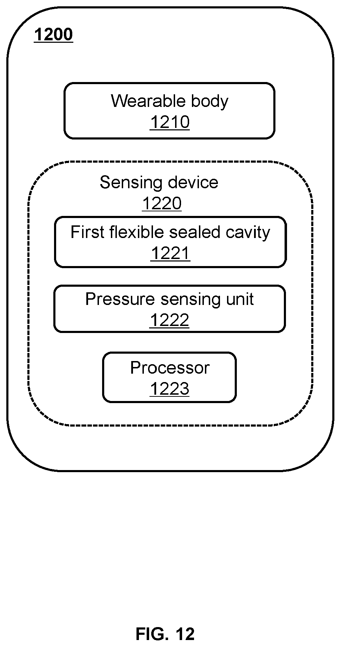

The sensing device shown in the various embodiments above may also be applied to various wearable devices. When the user wears the wearable device, the wearable device may detect a pressure exerted by the human body on the wearable device and/or detect a physiological signal of the user. Applications of the sensing device to wearable devices are illustrated exemplarily below in connection with FIG. 12 - FIG. 18.

FIG. 12 is a block diagram of a wearable device according to some embodiments of the present disclosure.

As shown in FIG. 12, a wearable device 1200 may include a wearable body 1210 and a sensing device 1220.

The wearable device 1200 refers to a device that may be worn by a user. In some embodiments, the wearable device 1200 may be worn on a body part such as the head, a wrist, etc., of the user. In some embodiments, the wearable device 1200 may include glasses, a smart bracelet, a finger ring, a headset, a hearing aid, a smart helmet, a smart watch, a smart garment, a smart backpack, a smart accessory, or the like, or any combinations thereof. For example, the wearable device 1200 may be functional myopia glasses, presbyopia glasses, cycling glasses, or sunglasses, etc., or it may be intelligent glasses, for example, audio glasses with headphone function. In some embodiments, the wearable device 1200 may also include head-mounted devices such as a helmet, an AR device, a VR device, etc. In some embodiments, the AR device or the VR device may include a VR helmet, VR glasses, an AR helmet, AR glasses, etc., or any combination thereof. For example, the VR device and/or the AR device may include Google Glass, Oculus Rift, Hololens, Gear VR, or the like.

The wearable body 1210 may be configured to be worn on the body of the user. In some embodiments, the wearable body 1210 may be an article of clothing (e.g., a T-shirt, a vest, a tank top, a jacket, etc.) worn on the upper half of the body of the user. In some embodiments, the wearable body 1210 may be a wristband, a watch, a ring, an armlet, a strap, etc., worn on a limb of the user. In some embodiments, the wearable body 1210 may also be a leg band or a belt, worn on a leg or the waist of the user, respectively. In some embodiments, the wearable body 1210 can also be an eyeglass frame, a headband, etc., worn on the head of the user. In some embodiments, the wearable body 1210 may be an object (e.g., clothing, a suction cup of an ECG monitor, etc.)

The sensing device 1220 may include a first flexible sealed cavity 1221, a pressure sensing unit 1222, and a processor 1223. In some embodiments, the sensing unit 1220 may be integrated on the wearable body 1210.

In some embodiments, the sensing device 1220 may be used to detect wearing pressure between the wearable device 1200 and the body of the user. In some embodiments, the first flexible sealed cavity 1221 may be arranged at a position where the wearable body 1210 contacts the body of the user. When the user wears the wearable device 1200, the wearing pressure between the wearable device 1200 and the body of the user may cause the first flexible sealed cavity 1221 to deform. The deformation of the first flexible sealed cavity 1221 may cause a change in the pressure within the first flexible sealed cavity 1221, and the pressure sensing unit 1222 may generate an electrical signal based on the change in the pressure. The processor 1223 may receive the electrical signal to determine the wearing pressure. It should be noted that the first flexible sealed cavity 1221 is similar to the flexible sealed cavity described above. The principle and structure of the sensing device 1220 for detecting the wearing pressure between the wearable device 1200 and the body of the user are similar to the principle and structure for detecting the wearing pressure between the acoustic output device and a facial region of the user. More descriptions of the sensing device 1220 may be found in FIG.1 - FIG.11 B and the related descriptions thereof.

In some embodiments, a flexible sealed structure (e.g., the first flexible sealing cavity 1221) of the sensing device 1220 may be configured to detect a physiological signal of the user. In some embodiments, the physiological signal may include, but is not limited to, a pulse, a heart rate, or a respiratory rate. In some embodiments, the first flexible sealed cavity 1221 may be provided in a specific region (e.g., a region where the physiological signal may be detected) where the wearable body 1210 is in contact with the body of the user. When the user wears the wearable device 1200, the first flexible sealed cavity 1221 is in contact with the body of the user, and the pulse, the heart rate, or the respiratory rate of the user may cause a change in the pressure within first flexible sealed cavity 1221, thereby causing the pressure sensing unit 1222 to generate an electrical signal, and the processor 1223 may identify a physiological signal based on the electrical signal. Specifically, the processor 1223 may record a cycle (time) of the electrical signal, and the cycle (time) of the electrical signal may reflect the heart rate, the pulse, or the respiratory rate of the user so as to realize the detection of the heart rate, the pulse, or the respiratory rate of the user.

In some embodiments, the sensing device 1220 having the first flexible sealed cavity 1221 may also be configured to detect the wearing pressure between the wearable device 1200 and the body of the user. In some embodiments, a plurality of sensing devices may be provided, wherein at least one first flexible sealed cavity 1221 may be configured to detect the wearing pressure between the wearable device 1200 and the body of the user. Further, the sensing device 1220 may also include a second flexible sealed cavity, the second flexible sealed cavity may be provided in a specific region where the wearable body 1210 is in contact with the body of the user. When the user wears the wearable device 1200, the second flexible sealed cavity is in contact with the body of the user, and the heart rate, the pulse, or the respiratory rate of the user may cause a change in the pressure inside the second flexible sealed cavity, which makes the pressure sensing unit 1222 generate an electrical signal. The processor 1223 may record an interval time (i.e., the cycle) between the generation of the electrical signals, thereby realizing the detection of the heart rate, the pulse, or the respiration rate of the user. The sensing device 1220 configured in this way may simultaneously detect the wearing pressure between the wearable device 1200 and the body of the user and detect the physiological signal of the user. If the sensing device 1220 includes the first flexible sealed cavity 1221 and the second flexible sealed cavity, two pressure sensing units 1222 may be provided within the first flexible sealed cavity 1221 and the second flexible sealed cavity, respectively, and the electrical signal generated by the pressure sensing unit 1222 of the first flexible sealed cavity 1221 and the electrical signal generated by the pressure sensing unit 1222 of the second flexible sealed cavity may be sent to a same processor ( the processor 1223) or different processors for processing.

The sensing device 1220 shown in FIG. 12 may be applied to various types of wearable devices 1200. The application of the sensing device 1220 in the exemplary wearable device 1200 is described below in conjunction with FIG.13 - FIG.18. The configuration of the sensing device 1220 in wearable device 1200 not shown in the drawings (e.g., a strap) is similar to the configurations shown in FIG.13 - FIG.18. It should be notes that FIG.13 - FIG.18 illustrate the position of the first flexible sealed cavity 1221 included in the sensing device 1220, with the pressure sensing unit 1222 and processor 1223 integrated within the wearable body 1210.

FIG. 13 is a schematic diagram of a structure of a smart bracelet according to some embodiments of the present disclosure. As shown in FIG. 13, a smart bracelet 1300 may include a banded structure 1311 and a smart bracelet body 1312. The sensing device may be integrated inside the smart bracelet 1300, and a portion of the smart bracelet 1300 forming the first flexible sealed cavity and/or the second flexible sealed cavity may be a portion of a surface region of the smart bracelet 1300, which is defined as a flexible region 1321 for ease of illustration. In some embodiments, the flexible region 1321 may seamlessly integrate with other regions of the surface of the smart bracelet 1300. In some embodiments, the flexible region 1321 may be set slightly protruding from other regions of the surface of the smart bracelet 1300. In some embodiments, the flexible region 1321 may also be integrated with other regions of the surface of the smart bracelet 1300.

In some embodiments, the flexible region 1321 of the smart bracelet 1300 may be disposed on a side of the banded structure 1311 that is in contact with the body of a user for detecting a wearing pressure of the banded structure 1311 against the wrist of the user or for monitoring a physiological signal of the user. In some embodiments, the flexible region 1321 of the smart bracelet 1300 may be disposed on a side of the smart bracelet body 1312 that is in contact with the body of the user. In some embodiments, two flexible regions 1321 may be provided on the side of the banded structure 1311 that is close to the body of the user and on the side of the smart bracelet body 1312 that is close to the body of the user, respectively. One of the two flexible regions 1321 may be configured to detect the wearing pressure between the banded structure 1311 and the wrist of the user, and another one of the two flexible regions 1321 may be configured to monitor the physiological signal of the user. In some embodiments, to allow the flexible region 1321 to detect the pressure between the band-like structure 1311 and the wrist of the user across a wider range, the flexible region 1321 may be arranged along an extension direction of the banded structure 1311.

If the wearing pressure between the smart bracelet 1300 and the body of the user is too small, it may interfere with the acquisition of the physiological signal of the user by the smart bracelet 1300. Therefore, in some embodiments, the processor may output a notification signal (e.g., a light signal, a beep signal, etc.) to the user in response to the wearing pressure falling below a preset contact force threshold to remind the user to adjust the wearing of the smart bracelet 1300. In some embodiments, the banded structure 1311 may be a length-adjustable structure (e.g., a buckle structure, a slot structure, etc.), and when the wearing pressure is lower than the preset contact force threshold, the wearing pressure between the smart bracelet 1300 and the user may be increased by adjusting a length of the banded structure 1311 shorter.

FIG. 14 is a schematic diagram of a structure of an ECG monitor according to some embodiments of the present disclosure. As shown in FIG. 14, an ECG monitor 1400 may include a suction cup 1412. The configuration of the sensing device on the ECG monitor 1400 may be similar to the configuration of the sensing device on the smart bracelet 1300. In some embodiments, a flexible region 1421 of the ECG monitor 1400 may be provided on a side of the suction cup 1412 near the body of a user. The ECG monitor 1400 may be configured to detect a wearing pressure of the suction cup against the body of the user and/or monitor a physiological signal of the user via the flexible region 1421 on the suction cup. In some embodiments, the ECG monitor 1400 may include a branded structure 1411, the branded structure 1411 may secure the ECG monitor 1400 near the chest of the user, and the flexible region 1421 may be arranged along an extension direction of the banded structure 1411.

FIG. 15 is a schematic diagram of a structure of glasses according to some embodiments of the present disclosure. As shown in FIG. 15, glasses 1500 may include a lens leg 1511 and a lens 1512. The lens 1512 may be connected to an end of the lens leg 1511, and a sensing device may be disposed on the glasses 1500 in a manner similar to the way the sensing device is provided on the smart bracelet 1300. In some embodiments, a flexible region 1521 of the glasses 1500 may be provided on a side of the lens leg 1511 that is away from the lens 1512, so that when the user wears the glasses 1500, the side of the lens legs 1511 that is away from the lens 1512 may be in contact with the head of the user and hang over an auricle of the user. The glasses 1500 may be configured to detect a wearing pressure of the lens leg 1511 against the head of the user and/or monitor a physiological signal of the user via the flexible region 1521 on the lens leg 1511.

In some embodiments, the flexible region 1521 may be disposed on a single lens leg 1511 when a count of the flexible region 1521 is one. In some embodiments, when the count of the flexible region 1521 is two, the two flexible regions 1521 may be disposed on two lens legs 1511, respectively. The flexible region(s) 1521 may be configured to detect a wearing pressure between the lens leg(s) 1511 and the head of the user, and may also be configured to monitor a physiological signal (e.g., a heart rate signal) of the user. In some embodiments, to enable the flexible region(s) 1521 to detect the pressure between the lens leg(s) 1511 and the head of the user over a wider range, the flexible region(s) 1521 may be disposed along an extension direction of the corresponding lens leg 1511.

In some embodiments, the glasses 1500 may be glasses 1500 with an audio function, and the lens leg 1511 may be provided with a speaker, a hearing aid, etc. Specifically, when the user wears the glasses 1500, the lens 1512 is located on the face of the user, the lens leg 1511 may extend from a side of the lens 1512 to attach to and support an ear of the user. The speaker or the hearing aid on the lens leg 1511 may be placed near the ear of the user. In some embodiments, to facilitate the installation of the speaker or the hearing aid, each lens leg 1511 may have a recessed structure on which the speaker or the hearing aid may be installed.

FIG. 16 is a schematic diagram of a structure of a virtual reality device or augmented reality device according to some embodiments of the present disclosure. As shown in FIG. 16, a virtual reality device or augmented reality device 1600 may include a rear hook 1611 and a device body 1612, two ends of the rear hook 1611 may be connected to the device body 1612, the device body 1612 may be a virtual reality device and/or an augmented reality device, etc., fixed to an end portion of the rear hook 1611. In some embodiments, the rear hook 1611 may secure the device body 1612 to the eyes of the user, and the rear hook 1611 may be secured around the head of the user when the user wears the virtual reality device or augmented reality device 1600. A sensing device may be provided on the virtual reality device or augmented reality device 1600 in a similar manner as the sensing device is provided on the smart bracelet 1300. In some embodiments, a flexible region 1621 of the virtual reality device or augmented reality device 1600 may be provided on the rear hook 1611. When the user wears the virtual reality device or augmented reality device 1600, the rear hook 1611 may deform and, at the same time, cause the flexible region 1621 to deform, such that the sensing device inside the flexible region 1621 may detect a pressure acting on the rear hook 1611 when the user wears the virtual reality device or augmented reality device 1600. When the user wears the virtual reality device or augmented reality device 1600 illustrated in FIG. 16, the flexible region 1621 may be disposed at a position on the rear hook 1611 near a back side of the head of the user, where the rear hook 1611 has a relatively large deformation. In some embodiments, the flexible region 1621 may also be disposed at a position on the device body 1612 in contact with a facial region of the user. In some embodiments, a count of the flexible region 1621 may be one or more, and the positions of the one or more the flexible regions 1621 on the rear hook 1611 may be found in FIG. 7 and the related descriptions thereof.

In some embodiments, the virtual reality device or augmented reality device 1600 may have an audio function, and a speaker, a hearing aids, etc., may be provided on the rear hook 1611. In some embodiments, the speaker or the hearing aid provided on the rear hook 1611 may be arranged close to an ear of the user. In some embodiments, to facilitate the installation of the speaker or the hearing aid, the rear hook 1611 may be provided with a recessed structure near the ear where the speaker or the hearing aid is installed.

FIG. 17 is a schematic diagram of a structure of a smart ring according to some embodiments of the present disclosure. As shown in FIG. 17, a smart ring 1700 may include a ring body 1720 and a circuit structure 1710. The ring body 1720 may be configured as a ring-shaped structure, and the circuit structure 1710 may be disposed on an outer ring side wall of the ring body 1720. A sensing device may be provided on the smart ring 1700 in a similar manner as the sensing device is provided on the smart bracelet 1300. In some embodiments, a flexible region 1721 of the smart ring 1700 may be provided on an inner ring side wall of the ring body 1720. The smart ring 1700 may be configured to detect a wearing pressure between the smart ring 1700 and a finger of the user and/or monitor a physiological signal of the user via the flexible region 1721 on the inner ring. In some embodiments, the circuit structure 1710 may be configured to provide power to the sensing device, receive and process the wearing pressure detected by the sensing device, and/or monitor the physiological signal of the user. In some embodiments, the circuit structure 1710 may also include a communication module, wherein the communication module may be configured to transmit information such as the wearing pressure and/or the physiological signal of the user to a user terminal (e.g., a cell phone, a computer, a smartwatch, etc.) in a wireless manner. In some embodiments, in order to more accurately detect the overall wearing pressure between the smart ring 1700 and the finger of the user, the flexible region 1721 may be configured as an annular structure, and the annular structure may be arranged circumferentially along the inner ring side wall of the smart ring 1700.

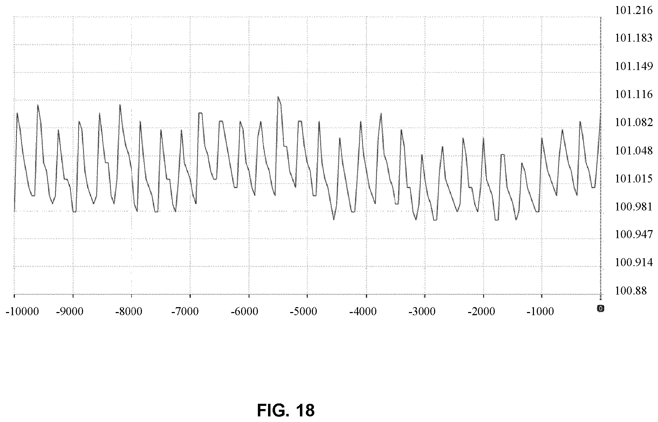

In some embodiments, the smart ring 1700 may monitor the physiological signal of the user through the flexible region 1721 on the inner ring side wall of the smart ring 1700. FIG. 18 is a schematic diagram of a curve of heart rate signals according to some embodiments of the present disclosure. FIG. 18 shows the heart rate signals measured by the sensing device when a second flexible cavity is provided on the inner ring side wall of the smart ring 1700, wherein the human heartbeat frequency is about 71 Hz.

It should be noted that the descriptions of FIG.1 - FIG.18 are provided for illustrative purposes and do not constitute any limitation of the disclosure. For a person of ordinary skill in the art, a variety of variations and modifications may be made in accordance with the scope of the appended claims. Different embodiments may produce different beneficial effects, and the beneficial effects that may be achieved in different embodiments may be any combination of the aforementioned or other possible beneficial effects.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, the scope of the invention however is defined in the appended claims.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims define the scope of the claimed invention. For example, although the implementation of various components described above may be embodied in a hardware device, it may also be implemented as a software only solution, e.g., an installation on an existing server or mobile device.

It should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This way of disclosure, however, is not to be interpreted as reflecting an intention that the claimed subject matter requires more features than are expressly recited in each claim. Rather, inventive embodiments as defined in the appended claims lie in less than all features of a single foregoing disclosed embodiment.

In some embodiments, the numbers expressing quantities or properties used to describe and claim certain embodiments of the present disclosure are to be understood as being modified in some instances by the term "about," "approximate," or "substantially." For example, "about," "approximate," or "substantially" may indicate $\pm 20\%$ variation of the value it describes, unless otherwise stated. Accordingly, in some embodiments, the numerical parameter set forth in the written description and attached claims are approximations that may vary depending upon the desired properties sought to be obtained by a particular embodiment. In some embodiments, the numerical parameter should be construed in light of the number of reported significant digits and by applying ordinary rounding techniques. Notwithstanding that the numerical ranges and parameter setting forth the broad scope of some embodiments of the present disclosure are approximations, the numerical values set forth in the specific examples are reported as precisely as practicable.

In closing, it is to be understood that the embodiments of the present disclosure disclosed herein are illustrating the invention and which is defined in the appended claims.

**Claims**

1. An acoustic output device (100), comprising an acoustic output unit (110), an ear hook (120), a pressure sensing unit (132), a processor (133), and an adjustment component (140), wherein

   the ear hook (120) is configured to suspend the acoustic output unit (110) near an ear of a user, and the ear hook (120) is provided with a volume-deformable flexible sealed cavity (131) configured to detect a pressure exerted on the ear hook (120) when the user wears the acoustic output device (100),
   the flexible sealed cavity (131) is in fluid communication with the pressure sensing unit (132), and the pressure sensing unit (132) generates an electrical signal based on a change in the pressure within the flexible sealed cavity (131); and
   the processor (133) is configured to determine the pressure exerted on the ear hook (120) when the user wears the acoustic output device (100) based on the electrical signal,
   the processor (133) is further configured to determine whether the pressure is within a preset pressure range and control the adjustment component (140) to adjust deformation of the ear hook (120), so that the pressure of the ear hook (120) is within the preset pressure range.

2. The acoustic output device (100) of claim 1, wherein the flexible sealed cavity (131) extends along an extension direction of the ear hook (120), the ear hook (120) is provided with an accommodation cavity (521) juxtaposed with the flexible sealed cavity (131), and a linear structure is arranged inside the accommodation cavity (521).

3. The acoustic output device (100) of claim 2, wherein the flexible sealed cavity (131) and the accommodation cavity (521) are integrally formed.

4. The acoustic output device (100) of claim 1, wherein the pressure sensing unit (132) is disposed in a closed cavity formed by the flexible sealed cavity (131), and the pressure sensing unit (132) converts the change in the pressure into the electrical signal in response to detecting the change in the pressure of fluid within the closed cavity.

5. The acoustic output device (100) of claim 1, wherein the processor (133) determines the pressure exerted on the ear hook (120) when the user wears the acoustic output device (100) based on the electrical signal and a machine learning model.

6. The acoustic output device (100) of claim 1, wherein the determining the pressure exerted on the ear hook (120) when the user wears the acoustic output device (100) based on the electrical signal includes:

   obtaining a calibration curve, wherein the calibration curve is obtained by fitting electrical signals generated by applying different preset loads to the flexible sealed cavity (131); and
   determining the pressure based on the calibration curve.

7. The acoustic output device (100) of claim 1, wherein the processor (133) is further configured to identify whether the user is wearing the acoustic output device (100) based on the pressure.

8. The acoustic output device (100) of claim 1, wherein the processor (133) is configured to determine a contact force between the acoustic output unit (110) and a skin of the user near the ear based on the pressure.

9. The acoustic output device (100) of claim 1, wherein the adjustment component (140) includes a piezoelectric element, the piezoelectric element is arranged along the extension direction of the ear hook (120), and the processor (133) is configured to control deformation of the piezoelectric element, so that the pressure of the ear hook (120) is within the preset pressure range.

10. The acoustic output device (100) of claim 1, further comprising a micro motor, wherein the ear hook (120) and the acoustic output unit (110) are rotatably connected through the adjustment component (140), and an output end of the micro motor is connected with the adjustment component (140).

11. The acoustic output device (100) of claim 1, further comprising a micro motor configured to drive an angular adjustment between the ear hook (120) and the acoustic output unit (110).

12. The acoustic output device (100) of any one of claims 1-11, wherein the flexible sealed cavity (131) includes a flexible cavity (3311), a connecting cavity (3312), and a connecting pipe (3313),

the flexible cavity (3311), the connecting cavity (3312), and the connecting pipe (3313) together form a closed cavity of the flexible sealed cavity (131),
the flexible cavity (3311) is configured to deform under an external load and produce the change in the pressure,
the connecting cavity (3312) accommodates the pressure sensing unit (132), and the connecting pipe (3313) connects the flexible cavity (3311) and the connecting cavity (3312).

13. The acoustic output device (100) of any one of claims 1-12, further comprising a rear hook (750), wherein

the ear hook (120) includes a first ear hook (722) and a second ear hook (723), the acoustic output unit (110) includes a first acoustic output unit (711) and a second acoustic output unit (712), the first ear hook (722) is connected with the first acoustic output unit (711) and the rear hook (750), the second ear hook (723) is connected with the second acoustic output unit (712) and the rear hook (750),
the first ear hook (722) suspends the first acoustic output unit (711) near one ear of the user, the second ear hook (723) suspends the second acoustic output unit (712) near the other ear of the user, and
the first ear hook (722) and the second ear hook (723) are connected via the rear hook (750).

**Patentansprüche**

1. Akustische Ausgabevorrichtung (100), umfassend eine akustische Ausgabeeinheit (110), einen Ohrbügel (120), eine Drucksensoreinheit (132), einen Prozessor (133) und eine Einstellkomponente (140), wobei

der Ohrbügel (120) dazu konfiguriert ist, die akustische Ausgabeeinheit (110) in der Nähe eines Ohrs des Benutzers zu halten, und der Ohrbügel (120) mit einem volumenverformbaren flexiblen abgedichteten Hohlraum (131) versehen ist, welcher dazu konfiguriert ist, einen auf den Ohrbügel (120) ausgeübten Druck zu erkennen, wenn der Benutzer die akustische Ausgabevorrichtung (100) trägt,
der flexible abgedichtete Hohlraum (131) in strömungstechnischer Kommunikation mit der Drucksensoreinheit (132) steht, und die Drucksensoreinheit (132) ein elektrisches Signal basierend auf einer Änderung des Drucks innerhalb des flexiblen abgedichteten Hohlraums (131) erzeugt; und
der Prozessor (133) dazu konfiguriert ist, basierend auf dem elektrischen Signal den auf den Ohrbügel (120) ausgeübten Druck zu bestimmen, wenn der Benutzer die akustische Ausgabevorrichtung (100) trägt,
der Prozessor (133) weiter dazu konfiguriert ist, zu bestimmen, ob der Druck innerhalb eines voreingestellten Druckbereichs liegt, und die Einstellkomponente (140) zu steuern, um die Verformung des Ohrbügels (120) so einzustellen, dass der Druck des Ohrbügels (120) innerhalb des voreingestellten Druckbereichs liegt.

2. Akustische Ausgabevorrichtung (100) nach Anspruch 1, wobei sich der flexible abgedichtete Hohlraum (131) entlang einer Erstreckungsrichtung des Ohrbügels (120) erstreckt, der Ohrbügel (120) mit einem Aufnahmehohlraum (521) versehen ist, welcher dem flexiblen abgedichteten Hohlraum (131) gegenüberliegt, und eine lineare Struktur innerhalb des Aufnahmehohlraums (521) angeordnet ist.

3. Akustische Ausgabevorrichtung (100) nach Anspruch 2, wobei der flexible abgedichtete Hohlraum (131) und der Aufnahmehohlraum (521) einstückig ausgebildet sind

4. Akustische Ausgabevorrichtung (100) nach Anspruch 1, wobei die Drucksensoreinheit (132) in einem geschlossenen Hohlraum eingerichtet ist, welcher durch den flexiblen abgedichteten Hohlraum (131) gebildet wird, und die Drucksensoreinheit (132) die Änderung des Drucks als Reaktion auf Erkennen der Änderung des Flüssigkeitsdrucks innerhalb des geschlossenen Hohlraums in das elektrische Signal umwandelt.

5. Akustische Ausgabevorrichtung (100) nach Anspruch 1, wobei der Prozessor (133) basierend auf dem elektrischen Signal und einem Modell für maschinelles Lernen den auf den Ohrbügel (120) ausgeübten Druck bestimmt, wenn der Benutzer die akustische Ausgabevorrichtung (100) trägt.

6. Akustische Ausgabevorrichtung (100) nach Anspruch 1, wobei das Bestimmen des auf den Ohrbügel (120) ausgeübten Drucks, wenn der Benutzer die akustische Ausgabevorrichtung (100) trägt, basierend auf dem elektrischen Signal beinhaltet:

Ermitteln einer Kalibrierkurve, wobei die Kalibrierkurve durch Anpassen von elektrischen Signalen ermittelt wird, welche durch Anwenden verschiedener voreingestellter Belastungen auf den flexiblen abgedichteten Hohlraum (131) erzeugt werden; und
Bestimmen des Drucks basierend auf der Kalibrierkurve.

7. Akustische Ausgabevorrichtung (100) nach Anspruch 1, wobei der Prozessor (133) weiter dazu konfiguriert ist, basierend auf dem Druck zu identifizieren, ob der Benutzer die akustische Ausgabevorrichtung (100) trägt.

8. Akustische Ausgabevorrichtung (100) nach Anspruch 1, wobei der Prozessor (133) dazu konfiguriert ist, basierend auf dem Druck eine Kontaktkraft zwischen der akustischen Ausgabeeinheit (110) und einer Haut des Benutzers in der Nähe des Ohrs zu bestimmen.

9. Akustische Ausgabevorrichtung (100) nach Anspruch 1, wobei die Einstellkomponente (140) ein piezoelektrisches Element beinhaltet, das piezoelektrische Element entlang der Erstreckungsrichtung des Ohrbügels (120) angeordnet ist, und der Prozessor (133) dazu konfiguriert ist, Verformung des piezoelektrischen Elements zu steuern, sodass der Druck des Ohrbügels (120) innerhalb des voreingestellten Druckbereichs liegt.

10. Akustische Ausgabevorrichtung (100) nach Anspruch 1, weiter einen Mikromotor umfassend, wobei der Ohrbügel (120) und die akustische Ausgabeeinheit (110) über die Einstellkomponente (140) drehbar verbunden sind, und ein Ausgangsende des Mikromotors mit der Einstellkomponente (140) verbunden ist.

11. Akustische Ausgabevorrichtung (100) nach Anspruch 1, weiter einen Mikromotor umfassend, welcher dazu konfiguriert ist, eine Winkeleinstellung zwischen dem Ohrbügel (120) und der akustischen Ausgabeeinheit (110) zu steuern.

12. Akustische Ausgabevorrichtung (100) nach einem der Ansprüche 1-11, wobei der flexible abgedichtete Hohlraum (131) einen flexiblen Hohlraum (3311), einen Verbindungshohlraum (3312) und ein Verbindungsrohr (3313) beinhaltet,

der flexible Hohlraum (3311), der Verbindungshohlraum (3312) und das Verbindungsrohr (3313) zusammen einen geschlossenen Hohlraum des flexiblen abgedichteten Hohlraums (131) bilden,
der flexible Hohlraum (3311) so konfiguriert ist, dass er sich unter einer äußeren Last verformt und eine Änderung des Drucks erzeugt,
der Verbindungshohlraum (3312) die Drucksensoreinheit (132) aufnimmt, und das Verbindungsrohr (3313) den flexiblen Hohlraum (3311) mit dem Verbindungshohlraum (3312) verbindet.

13. Akustische Ausgabevorrichtung (100) nach einem der Ansprüche 1-12, weiter einen hinteren Bügel (750) umfassend, wobei

der Ohrbügel (120) einen ersten Ohrbügel (722) und einen zweiten Ohrbügel (723) beinhaltet, die akustische Ausgabeeinheit (110) eine erste akustische Ausgabeeinheit (711) und eine zweite akustische Ausgabeeinheit (712) beinhaltet, der erste Ohrbügel (722) mit der ersten akustischen Ausgabeeinheit (711) und dem hinteren Bügel (750) verbunden ist, der zweite Ohrbügel (723) mit der zweiten akustischen Ausgabeeinheit (712) und dem hinteren Bügel (750) verbunden ist,
der erste Ohrbügel (722) die erste akustische Ausgabeeinheit (711) in der Nähe eines Ohrs des Benutzers hält,
der zweite Ohrbügel (723) die zweite akustische Ausgabeeinheit (712) in der Nähe des anderen Ohrs des Benutzers hält, und
der erste Ohrbügel (722) und der zweite Ohrbügel (723) über den hinteren Bügel (750) miteinander verbunden sind.

**Revendications**

1. Dispositif de sortie acoustique (100), comprenant une unité de sortie acoustique (110), un crochet auriculaire (120), une unité de détection de pression (132), un processeur (133) et un composant d'ajustement (140), dans lequel

le crochet auriculaire (120) est configuré pour suspendre l'unité de sortie acoustique (110) près d'une oreille d'un utilisateur, et le crochet auriculaire (120) est doté d'une cavité étanche flexible déformable en volume (131) configurée pour détecter une pression exercée sur le crochet auriculaire (120) lorsque l'utilisateur porte le dispositif de sortie acoustique (100),

la cavité étanche flexible (131) est en communication fluidique avec l'unité de détection de pression (132), et l'unité de détection de pression (132) génère un signal électrique sur la base d'un changement de pression dans la cavité étanche flexible (131) ; et

le processeur (133) est configuré pour déterminer la pression exercée sur le crochet auriculaire (120) lorsque l'utilisateur porte le dispositif de sortie acoustique (100) sur la base du signal électrique,

le processeur (133) est en outre configuré pour déterminer si la pression se situe dans une plage de pression prédéfinie et commander le composant d'ajustement (140) pour ajuster la déformation du crochet auriculaire (120), de façon à ce que la pression du crochet auriculaire (120) se situe dans la plage de pression prédéfinie.

2. Dispositif de sortie acoustique (100) selon la revendication 1, dans lequel la cavité étanche flexible (131) s'étend le long d'une direction d'extension du crochet auriculaire (120), le crochet auriculaire (120) est doté d'une cavité de réception (521) juxtaposée à la cavité étanche flexible (131), et une structure linéaire est agencée à l'intérieur de la cavité de réception (521).

3. Dispositif de sortie acoustique (100) selon la revendication 2, dans lequel la cavité étanche flexible (131) et la cavité de réception (521) sont formées d'un seul bloc.

4. Dispositif de sortie acoustique (100) selon la revendication 1, dans lequel l'unité de détection de pression (132) est disposée dans une cavité fermée formée par la cavité étanche flexible (131), et l'unité de détection de pression (132) convertit le changement de pression en signal électrique en réponse à la détection du changement de pression de fluide dans la cavité fermée.

5. Dispositif de sortie acoustique (100) selon la revendication 1, dans lequel le processeur (133) détermine la pression exercée sur le crochet auriculaire (120) lorsque l'utilisateur porte le dispositif de sortie acoustique (100) sur la base du signal électrique et d'un modèle d'apprentissage automatique.

6. Dispositif de sortie acoustique (100) selon la revendication 1, dans lequel la détermination de la pression exercée sur le crochet auriculaire (120) lorsque l'utilisateur porte le dispositif de sortie acoustique (100) sur la base du signal électrique inclut :

l'obtention d'une courbe d'étalonnage, dans lequel la courbe d'étalonnage est obtenue en adaptant des signaux électriques générés par l'application de différentes charges prédéfinies à la cavité étanche flexible (131) ; et la détermination de la pression sur la base de la courbe d'étalonnage.

7. Dispositif de sortie acoustique (100) selon la revendication 1, dans lequel le processeur (133) est en outre configuré pour identifier si l'utilisateur porte le dispositif de sortie acoustique (100) sur la base de la pression.

8. Dispositif de sortie acoustique (100) selon la revendication 1, dans lequel le processeur (133) est configuré pour déterminer une force de contact entre l'unité de sortie acoustique (110) et la peau de l'utilisateur près de l'oreille sur la base de la pression.

9. Dispositif de sortie acoustique (100) selon la revendication 1, dans lequel le composant d'ajustement (140) inclut un élément piézoélectrique, l'élément piézoélectrique est agencé le long de la direction d'extension du crochet auriculaire (120), et le processeur (133) est configuré pour commander la déformation de l'élément piézoélectrique, de façon à ce que la pression du crochet auriculaire (120) se situe dans la plage de pression prédéfinie.

10. Dispositif de sortie acoustique (100) selon la revendication 1, comprenant en outre un micromoteur, dans lequel le crochet auriculaire (120) et l'unité de sortie acoustique (110) sont reliés de manière rotative par l'intermédiaire du composant d'ajustement (140), et une extrémité de sortie du micromoteur est reliée au composant d'ajustement (140).

11. Dispositif de sortie acoustique (100) selon la revendication 1, comprenant en outre un micromoteur configuré pour entraîner un ajustement angulaire entre le crochet auriculaire (120) et l'unité de sortie acoustique (110).

12. Dispositif de sortie acoustique (100) selon l'une quelconque des revendications 1-11, dans lequel la cavité étanche flexible (131) inclut une cavité flexible (3311), une cavité de liaison (3312) et un tuyau de liaison (3313),

la cavité flexible (3311), la cavité de liaison (3312) et le tuyau de liaison (3313) forment ensemble une cavité fermée de la cavité étanche flexible (131),

la cavité flexible (3311) est configurée pour se déformer sous une charge externe et produire le changement de pression,

la cavité de liaison (3312) reçoit l'unité de détection de pression (132), et le tuyau de liaison (3313) relie la cavité flexible (3311) et la cavité de liaison (3312).

13. Dispositif de sortie acoustique (100) selon l'une quelconque des revendications 1-12, comprenant en outre un crochet arrière (750), dans lequel

le crochet auriculaire (120) inclut un premier crochet auriculaire (722) et un second crochet auriculaire (723), l'unité de sortie acoustique (110) inclut une première unité de sortie acoustique (711) et une seconde unité de sortie acoustique (712), le premier crochet auriculaire (722) est relié à la première unité de sortie acoustique (711) et au crochet arrière (750), le second crochet auriculaire (723) est relié à la seconde unité de sortie acoustique (712) et au crochet arrière (750),

le premier crochet auriculaire (722) suspend la première unité de sortie acoustique (711) près d'une oreille de l'utilisateur, le second crochet auriculaire (723) suspend la seconde unité de sortie acoustique (712) près de l'autre oreille de l'utilisateur, et

le premier crochet auriculaire (722) et le second crochet auriculaire (723) sont reliés via le crochet arrière (750).

**FIG. 1**

FIG. 2

<u>**330**</u>

331

3312                    3313              3311

332

a

b

Extension direction

**FIG. 3**

**430**

431/3311

a

b

332

Extension direction

⟷

**FIG. 4**

**500**

B

A

520

510

**FIG. 5**

521    531

**FIG. 6**

<u>700</u>

FIG. 7

**800**

FIG. 8

900

FIG. 9

1000

1010

1011

1031

1020

**FIG.10A**

1000

1034

1010

1012

1020

**FIG.10B**

**1100**

1110

1131

1120

**FIG. 11A**

**1100**

1110

1111

1131

1120

**FIG. 11B**

**1200**

Wearable body
1210

Sensing device
1220

First flexible sealed cavity
1221

Pressure sensing unit
1222

Processor
1223

**FIG. 12**

1300

1311

1312

1321

FIG. 13

<u>1400</u>

FIG. 14

<u>1500</u>

1521

1511

1512

FIG. 15

1600

1611

1621

1612

FIG. 16

1700

FIG. 17

**FIG. 18**

**REFERENCES CITED IN THE DESCRIPTION**

**Patent documents cited in the description**

- CN 202211006787
- WO 2024088113 A1
- CN 113259808 A
- CN 211128122 U
- US 2018199127 A1
- WO 2016011848 A1